# EUROPEAN PATENT APPLICATION

(11) **EP 4 585 617 A1**
(43) Date of publication of application: **16.07.2025**
(21) Application number: 23862496.9
(22) Date of filing: 08.09.2023
(51) Int. Cl.: C07K 16/28, C07K 19/00, C07K 14/50, C12N 15/13, C12N 15/62, A61K 39/395, A61P 3/04, A61P 3/10

(54) **GIPR ANTIBODY, FUSION PROTEIN THEREOF WITH FGF21, PHARMACEUTICAL COMPOSITION THEREOF, AND USE THEREOF**

(30) Priority: 08.09.2022 CN 202211098122
(71) Applicant: Gmax Biopharm LLC, Hangzhou, Zhejiang 310052 (CN)
(72) Inventor: WANG, Xiaofeng, Hangzhou, Zhejiang 310052 (CN); ZHANG, Hua, Hangzhou, Zhejiang 310052 (CN); ZHANG, Cheng, Hangzhou, Zhejiang 310052 (CN); JING, Shuqian, Hangzhou, Zhejiang 310052 (CN)
(74) Representative: Simmons & Simmons LLP (Munich)
(86) International application number: PCT/CN2023/117637
(87) International publication number: WO 2024/051802

(57) **Abstract**

Provided are a GIPR antibody, a fusion protein thereof with FGF21, a pharmaceutical composition thereof, and use thereof. Further provided is a method of the GIPR antibody and the fusion protein thereof with FGF21 for treating, preventing or improving one or a plurality of symptoms of non-alcoholic fatty liver disease, non-alcoholic steatohepatitis, obesity, or Type 2 diabetes.

## Description

### Technical Field

Provided herein are a GIPR antibody and a fusion protein thereof with FGF21, as well as a pharmaceutical composition thereof. Further provided herein is a method of the GIPR antibody and the fusion protein thereof with FGF21 for treating, preventing or improving one or a plurality of symptoms of non-alcoholic fatty liver disease, non-alcoholic steatohepatitis, obesity, or Type 2 diabetes.

### Background Art

Gastric Inhibitory Polypeptide (GIP) is a polypeptide hormone, a polypeptide containing 42 amino acid residues, which is secreted by intestinal K cells in humans after eating. GIP is involved in the physiological process of stimulating insulin secretion by activating the Gastric Inhibitory Polypeptide Receptor (GIPR) on the surface of pancreatic beta cells (Tseng et al., 1996, J. Clin. Invest. 98:2440-2445; Ravn et al., 2013, J. Biol. Chem. 288:19760-72). In addition to being distributed in pancreas, GIPR is widely distributed, including bones, heart, stomach, intestinal tract and adipose tissue, etc. (Peter et al., 2013, J. Biol. Chem. 288:19760-72). The diverse distribution suggests that the GIP/GIPR pathway has more biological effects beyond blood glucose regulation. Experimental evidence shows that the GIP/GIPR signaling pathway is at least closely associated with fat metabolism in these tissues (Yip et al., 2000, Life Sci . 66: 91-103). Experimental data also show that the GIP concentration in the circulating blood of patients with obesity or diabetes is elevated (Creutzfeldt et al., 1978, Diabetologia 14:15-24; Flatt et al., 1984, J. Endocrinol. 101:249-256; Salera et al., 1982, J. Clin. Endocrinol. Metab. 55:329-336; Vilsbøll et al., 2003, J. Clin. Endocrinol. Metab. 88:2706-2713).

Fibroblast Growth Factor (FGF21) is an endogenous protein synthesized in the liver, is a member of the FGF family and has important functions in regulating glucose and lipid metabolism (Schlein et al., 2016, Cell Metabolism. 23:441-453; Habegger et al., 2013, Diabetes. 62:1453-1463). FGF21 can act on the liver, adipocytes, beta cells of the pancreatic islets, muscle tissues and the hypothalamus of the brain, and exerts its physiological function by activating an FGF21-specific receptor (Kharitonenkov et al., 2005, J. Clin. Invest. 115:1627-1635; Dunshe et al., 2016, J. Biol. Chem.291:5986-5996)*.* FGF21 can activate a receptor complex on the cell membrane, comprising β-Klotho and one of FGF receptors, FGFR1c, FGFR2c or FGFR3c. Expression of these receptors is tissue specific, for example, FGFR1c is predominantly expressed in adipose tissue, while FGFR2c and FGFR3c are predominantly expressed in the liver (Yie et al., 2012, Chem. Biol. Drug Des. 79:398-410). The concentration of endogenous FGF21 in adipose tissue is about 100 pg/mL in healthy humans, and can be elevated 10-fold in patients with obesity, or non-alcoholic steatohepatitis, which is predictive of the presence of FGF21 resistance in those patients. Dietary changes also affect the circulating level of FGF21 in humans, wherein heavy consumption of fructose or alcohol will dramatically increase the level of FGF21 in plasma; limiting protein intake for long periods of time or excessive intake of carbohydrates will increase FGF21 levels as well.

The fat-reducing effects of the GIPR antibody and the effects of FGF21 in reducing liver inflammation, fibrosis and improving lipoproteins are combined herein to treat non-alcoholic fatty liver disease, non-alcoholic steatohepatitis, obesity and Type 2 diabetes through these multiple effects. Provided herein is a fusion protein drug for use in treating those patients suffering from one or more diseases of non-alcoholic fatty liver disease, non-alcoholic steatohepatitis, obesity, and Type 2 diabetes.

### Summary of the Invention

Provided herein is an antibody capable of specifically binding to a GIPR, which is an antagonist of GIPR.

Also provided herein is an antibody capable of specifically binding to a GIPR, wherein the antibody comprises one, two, three, four, five, or six amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11;
b. light chain CDR2 amino acid sequences: YAS, SEQ ID NO: 2;
c. light chain CDR3 amino acid sequences: SEQ ID NO: 3 and SEQ ID NO: 4;
d. heavy chain CDR1 amino acid sequence: SEQ ID NO: 12;
e. heavy chain CDR2 amino acid sequence: SEQ ID NO: 13; and
f. heavy chain CDR3 amino acid sequence: SEQ ID NO: 14.

Further provided herein is an FGF21 fusion protein, wherein the fusion protein comprises an antibody capable of specifically binding to a GIPR, and one, two, three, four, five, six, seven, or eight FGF21 fragments; in the fusion protein, the amino terminus of an FGF21 fragment is linked to the carboxy terminus of a GIPR antibody light or heavy chain via a peptide linker sequence (Linker).

Provided herein is an FGF21 fusion protein, wherein the fusion protein comprises an antibody capable of specifically binding to a GIPR, and one, two, three, or four FGF21 fragments; in the fusion protein, the amino terminus of an FGF21 fragment is linked to the carboxy terminus of a GIPR antibody light or heavy chain via a peptide linker sequence (Linker).

Provided herein is an FGF21 fusion protein, wherein the fusion protein comprises an antibody capable of specifically binding to a GIPR, and one, or two FGF21 fragments; in the fusion protein, the amino terminus of an FGF21 fragment is linked to the carboxy terminus of a GIPR antibody light or heavy chain via a peptide linker sequence (Linker).

Provided herein is an FGF21 fusion protein, wherein the fusion protein comprises a GIPR antibody and one or two FGF21 fragments; in the fusion protein, the amino terminus of an FGF21 fragment is linked to the carboxy terminus of a GIPR antibody light chain via a peptide linker sequence (Linker): N'-R-Linker-FGF21-C'; or the amino terminus of an FGF21 fragment is linked to the carboxy terminus of a GIPR antibody heavy chain: N'-R-Linker-FGF21-C'; wherein N' represents the amino terminus of a polypeptide chain of the fusion protein, C' represents the carboxy terminus of a polypeptide chain of the fusion protein, FGF21 represents an FGF21 fragment, R is the amino acid sequence of the light or heavy chain of the GIPR antibody, and Linker represents a peptide linker sequence.

Provided herein is a polynucleotide encoding a GIPR antibody described herein.

Provided herein is a polynucleotide encoding a fusion protein of a GIPR antibody and FGF21 described herein.

Provided herein is a vector comprising a polynucleotide encoding a GIPR antibody described herein.

Provided herein is a vector comprising a polynucleotide encoding a fusion protein of a GIPR antibody and FGF21 described herein.

Provided herein is a host cell comprising a vector described herein.

Provided herein is a pharmaceutical composition comprising a GIPR antibody described herein and a pharmaceutically acceptable carrier.

Provided herein is a pharmaceutical composition comprising a fusion protein of a GIPR antibody and FGF21 described herein and a pharmaceutically acceptable carrier.

Provided herein is the use of a GIPR antibody described herein in the preparation of a medicament for treating, preventing or improving non-alcoholic steatohepatitis-related diseases.

Provided herein is the use of a fusion protein of a GIPR antibody and FGF21 described herein in the preparation of a medicament for treating, preventing or improving non-alcoholic steatohepatitis-related diseases.

Provided herein is the use of a GIPR antibody described herein in the preparation of a medicament for treating, preventing or improving Type 2 diabetes.

Provided herein is the use of a fusion protein of a GIPR antibody and FGF21 described herein in the preparation of a medicament for treating, preventing or improving Type 2 diabetes.

Provided herein is the use of a GIPR antibody described herein in the preparation of a medicament for weight loss or for treating, preventing or improving obesity and conditions associated with obesity.

Provided herein is the use of a fusion protein of a GIPR antibody and FGF21 described herein in the preparation of a medicament for weight loss or for treating, preventing or improving obesity and conditions associated with obesity.

Provided herein is the use of a GIPR antibody described herein in the preparation of a medicament for simultaneously treating, preventing or improving two or more conditions of non-alcoholic steatohepatitis-related diseases, obesity, or Type 2 diabetes.

Provided herein is the use of a fusion protein of a GIPR antibody and FGF21 described herein in the preparation of a medicament for simultaneously treating, preventing or improving two or more conditions of non-alcoholic steatohepatitis-related diseases, obesity, or Type 2 diabetes.

Provided herein is a method for treating, preventing, or improving one or more symptoms of non-alcoholic steatohepatitis-related diseases, comprising administering to a subject a therapeutically effective amount of a GIPR antibody described herein.

Provided herein is a method for treating, preventing, or improving one or more symptoms of non-alcoholic steatohepatitis-related diseases, comprising administering to a subject a therapeutically effective amount of a fusion protein of a GIPR antibody and FGF21 described herein.

Provided herein is a method for treating, preventing, or improving one or more symptoms of obesity, comprising administering to a subject a therapeutically effective amount of a GIPR antibody described herein.

Provided herein is a method for treating, preventing, or improving one or more symptoms of obesity, comprising administering to a subject a therapeutically effective amount of a fusion protein of a GIPR antibody and FGF21 described herein.

Provided herein is a method for treating, preventing, or improving one or more symptoms of Type 2 diabetes, comprising administering to a subject a therapeutically effective amount of a GIPR antibody described herein.

Provided herein is a method for treating, preventing, or improving one or more symptoms of Type 2 diabetes, comprising administering to a subject a therapeutically effective amount of a fusion protein of a GIPR antibody and FGF21 described herein.

### Brief Description of the Drawings

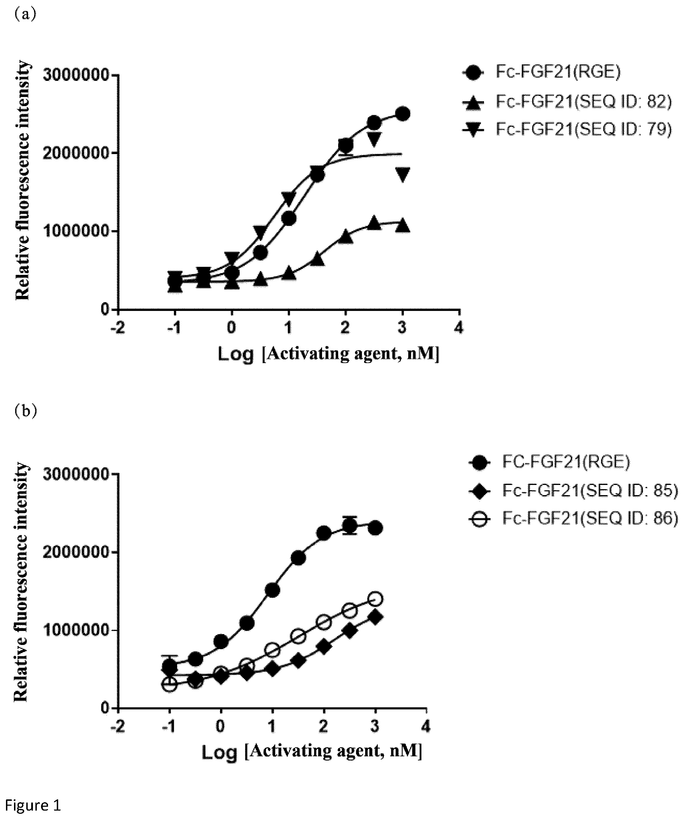

FIG. 1 shows activating curves of Fc-FGF21 fusion proteins Fc-linker-FGF21 (which comprise SEQ ID NO: 79, SEQ ID NO: 82, SEQ ID NO: 85 and SEQ ID NO: 86) for activating hFGFR1c/hKLB signaling pathway, as detected in a reporter gene experiment, wherein FIG. 1(a) shows that EC₅₀ is 18.61 nM for Fc-FGF21 (RGE), 5.57 nM for Fc-FGF21 (SEQ ID NO: 79), and 41.40 nM for Fc-FGF21 (SEQ ID NO: 82), respectively; and FIG. 1(b) shows that EC₅₀ is 8.13 nM for Fc-FGF21 (RGE), 156.50 nM for Fc-FGF21 (SEQ ID NO: 85), 31.44 nM for Fc-FGF21 (SEQ ID NO: 86), respectively.

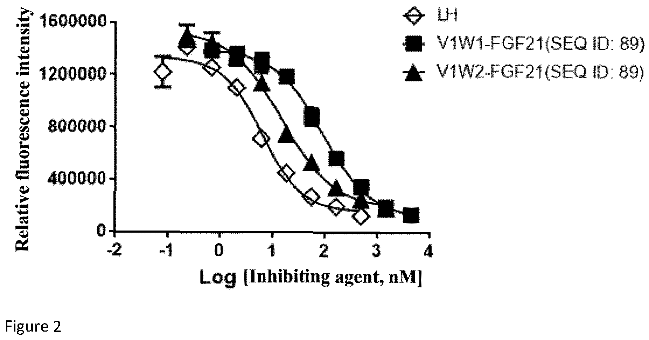

FIG. 2 shows concentration-inhibition curves of GIPR antibody/FGF21 fusion proteins V1W1-Linker-FGF21 (which comprises SEQ ID NO: 89), V1W2-Linker-FGF21 (which comprises SEQ ID NO: 89) for antagonizing the hGIPR signaling pathway activated by a GIP, as detected in a reporter gene experiment, with an IC₅₀ of 6.50 nM for LH, 91.14 nM for V1W1-FGF21 (SEQ: 89), and 15.30 nM for V1W2-FGF21 (SEQ: 89), respectively.

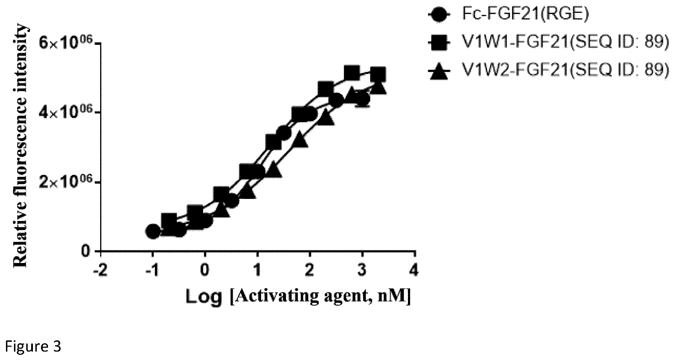

FIG. 3 shows activating curves of GIPR antibody/FGF21 fusion proteins V1W1-Linker-FGF21 (which comprises SEQ ID NO: 89), V1W2-Linker-FGF21 (which comprises SEQ ID NO: 89) for activating the hFGFR1C/hKLB signaling pathway, as detected in a reporter gene experiment, with EC₅₀ of 11.65 nM for Fc-FGF21 (RGE), 16.44 nM for V1W1-FGF21 (SEQ ID NO: 89), and 37.80 nM for V1W2-FGF21 (SEQ ID NO: 89), respectively.

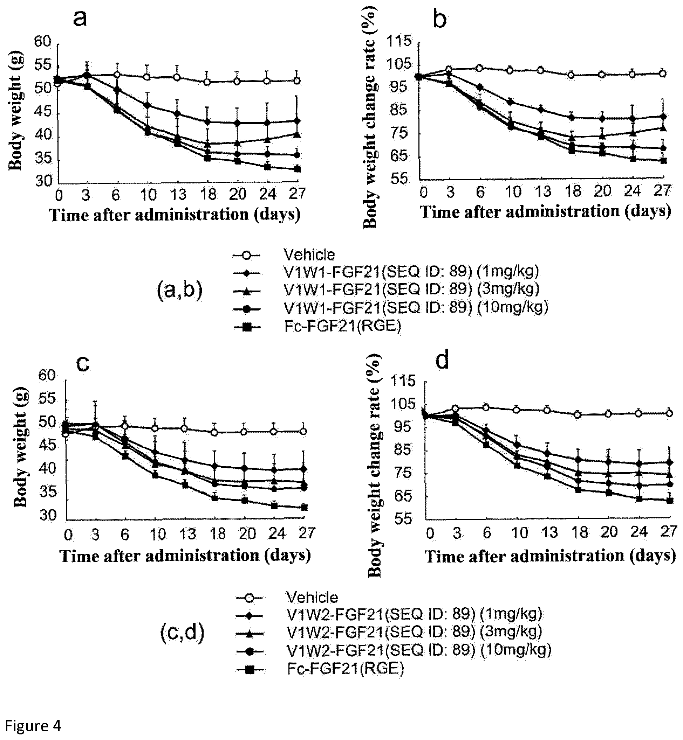

FIG. 4 shows curves of mouse body weight (a, c) and mouse body weight change rate (b, d) over time in high-fat feed-induced C57BL/6 obese mice within a cycle in a pharmacodynamic experiment with hGIPR antibody/FGF21 fusion proteins V1W1-Linker-FGF21 (which comprises SEQ ID NO: 89), V1W2-Linker-FGF21 (which comprises SEQ ID NO: 89) at high, medium, and low doses.

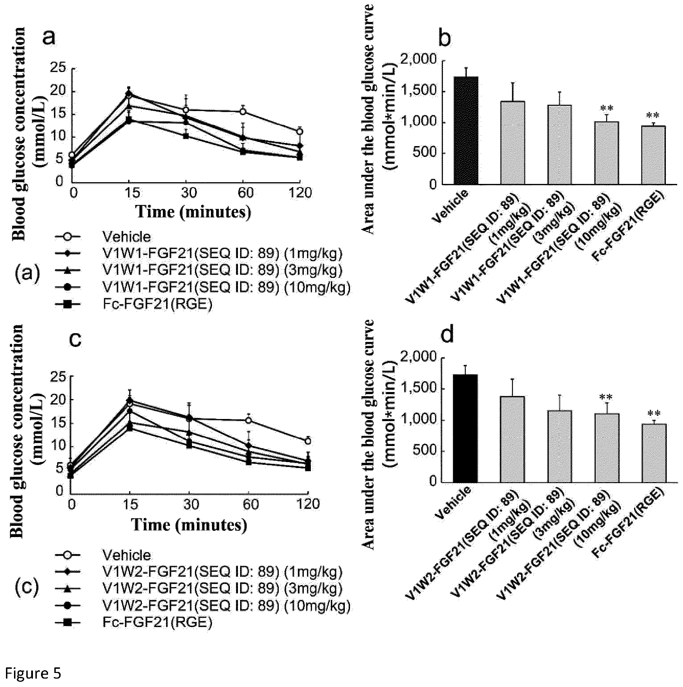

FIG. 5 shows curves of OGTT over time (a, c) and values of the area under the blood glucose curves (b, d) in high-fat feed-induced obese mice within a cycle in a pharmacodynamic experiment with hGIPR antibody/FGF21 fusion proteins V1W1-Linker-FGF21 (which comprises SEQ ID NO: 89), V1W2-Linker-FGF21 (which comprises SEQ ID NO: 89) at high, medium, and low doses.

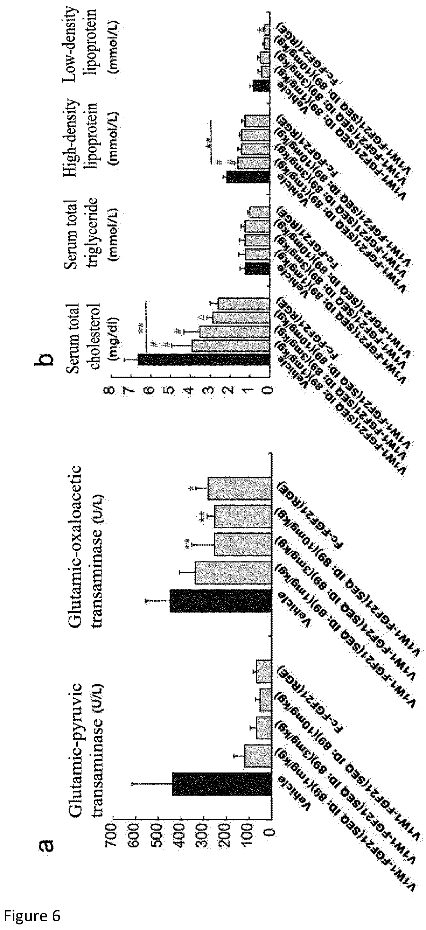

FIG. 6 shows the effects of high, medium, and low doses of the hGIPR antibody/FGF21 fusion protein V1W1-Linker-FGF21 (which comprises SEQ ID NO: 89) on serum indicators of liver function of mice (a) and serum indicators of lipid metabolism (b) in high-fat feed-induced obese mice within a cycle in a pharmacodynamic experiment.

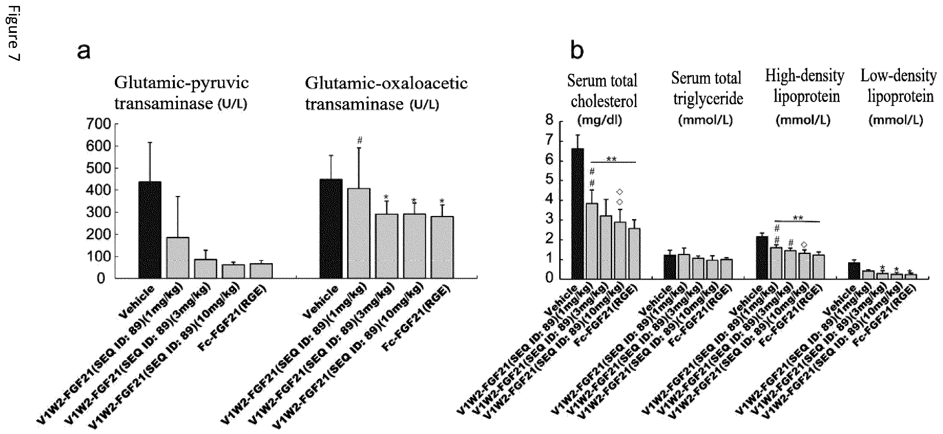

FIG. 7 shows the effects of high, medium, and low doses of the hGIPR antibody/FGF21 fusion protein V1W2-Linker-FGF21 (which comprises SEQ ID NO: 89) on serum indicators of liver function (a) and serum indicators of lipid metabolism (b) in high-fat feed-induced obese mice within a cycle in a pharmacodynamic experiment.

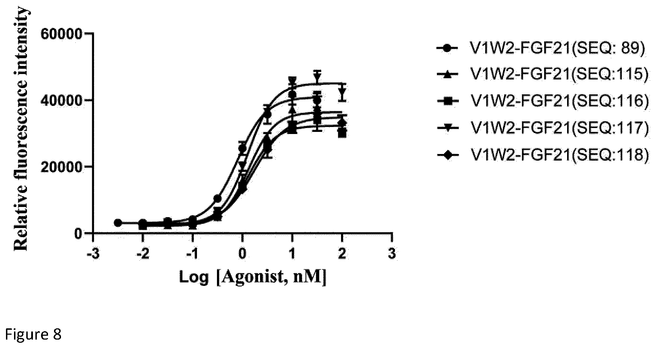

FIG. 8 shows activating curves of GIPR antibody/FGF21 fusion proteins V1W2-Linker-FGF21 (which comprises SEQ: 89), V1W2-Linker-FGF21 (which comprises SEQ ID NO: 115), V1W2-Linker-FGF21 (which comprises SEQ ID NO: 116), V1W2-Linker-FGF21 (which comprises SEQ ID NO: 117), V1W2-Linker-FGF21 (which comprises SEQ ID NO: 118) for activating the mFGFR1C/hKLB signaling pathway, as detected in a reporter gene experiment, wherein EC₅₀ is 0.80 nM for V1W2-FGF21 (SEQ: 89), 1.33 nM for V1W2-FGF21 (SEQ: 115), 1.35 nM for V1W2-FGF21 (SEQ: 116), 1.29 nM for V1W2-FGF21 (SEQ: 117) and 1.71 nM for V1W2-FGF21 (SEQ: 118), respectively.

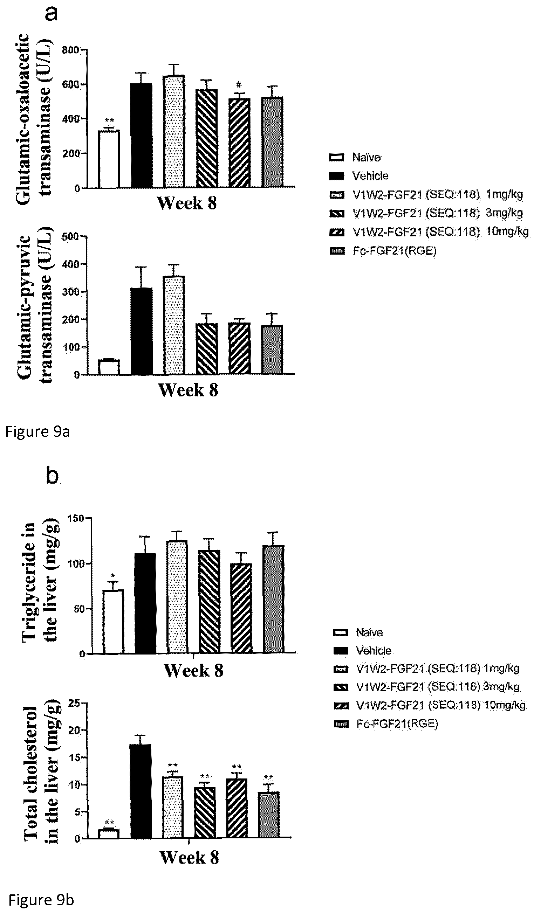

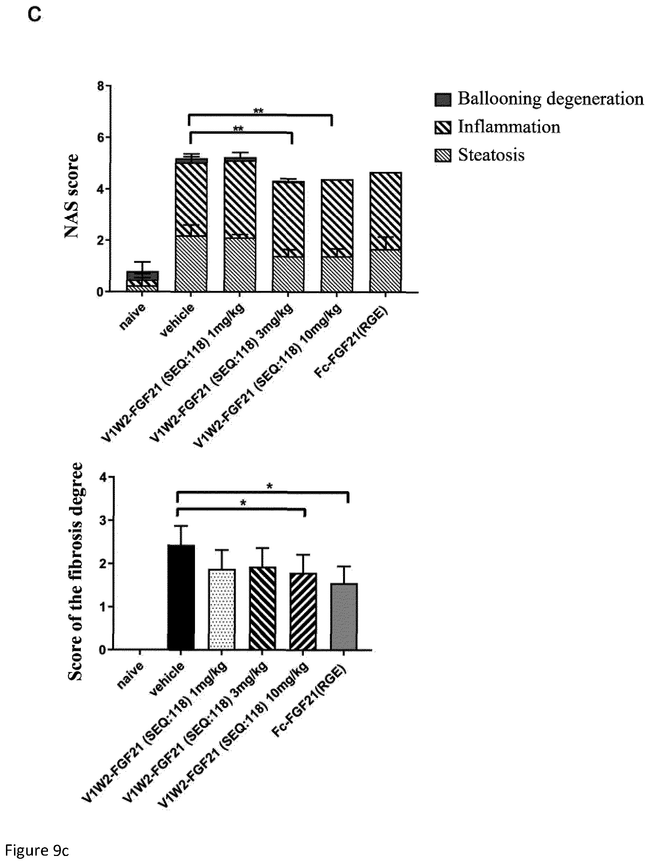

FIG. 9 shows the effects of high, medium, and low doses of the hGIPR antibody/FGF21 fusion protein V1W2-Linker-FGF21 (which comprises SEQ ID NO: 118) on serum indicators of liver function (a), serum indicators of lipid metabolism (b) and NAS score and fibrosis score (c) in mice with high-fat feed-induced non-alcoholic steatohepatitis within a cycle in a pharmacodynamic experiment.

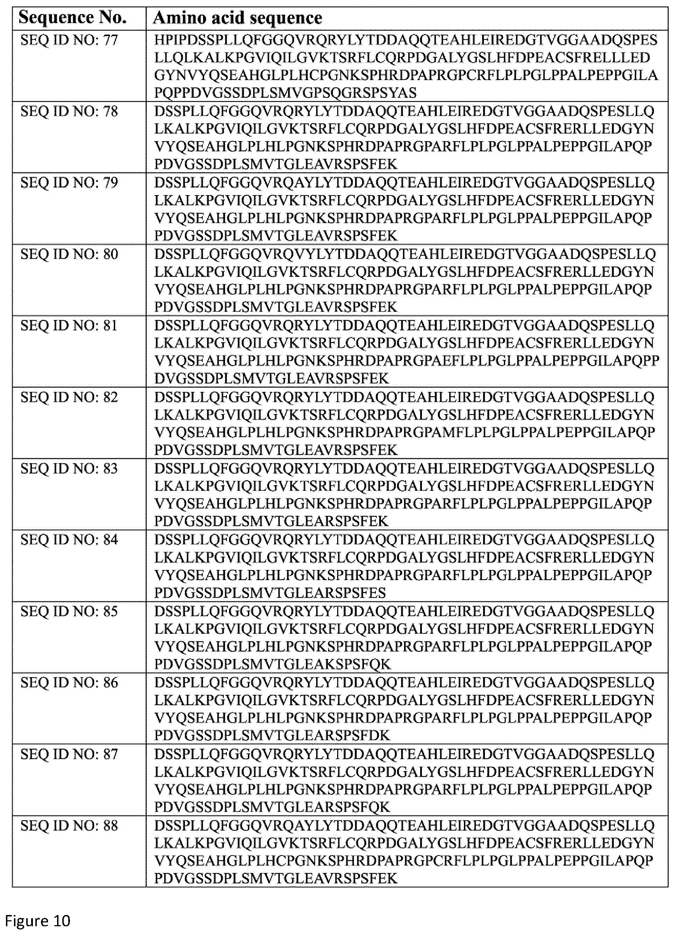

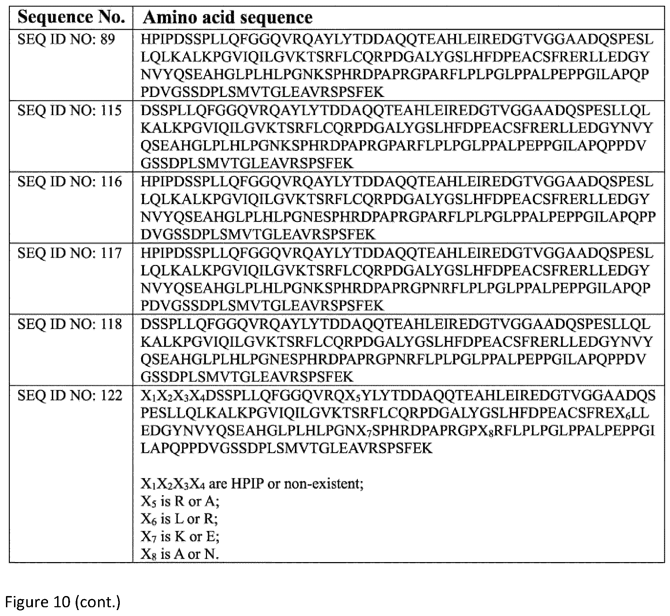

FIG. 10 shows the variant sequences of a wild-type FGF21, as set forth in SEQ ID NO: 77 to SEQ ID NO: 89, SEQ ID NO: 115 to SEQ ID NO: 118, and SEQ ID NO: 122.

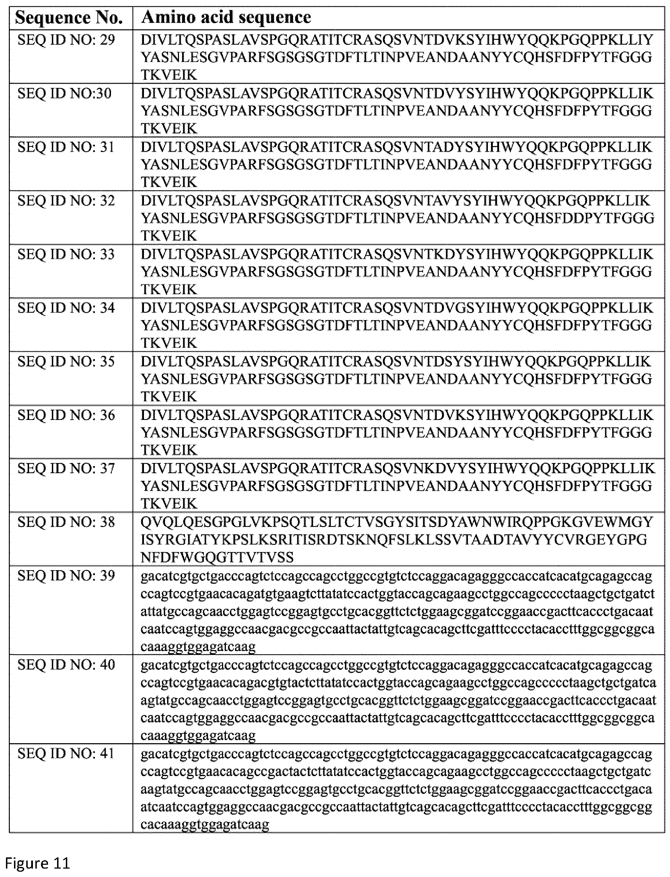

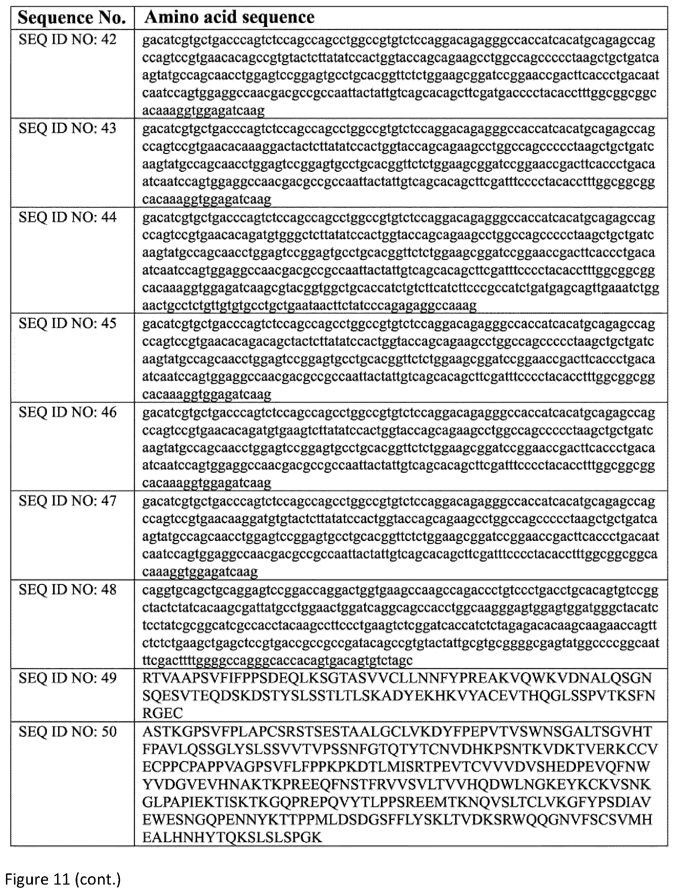

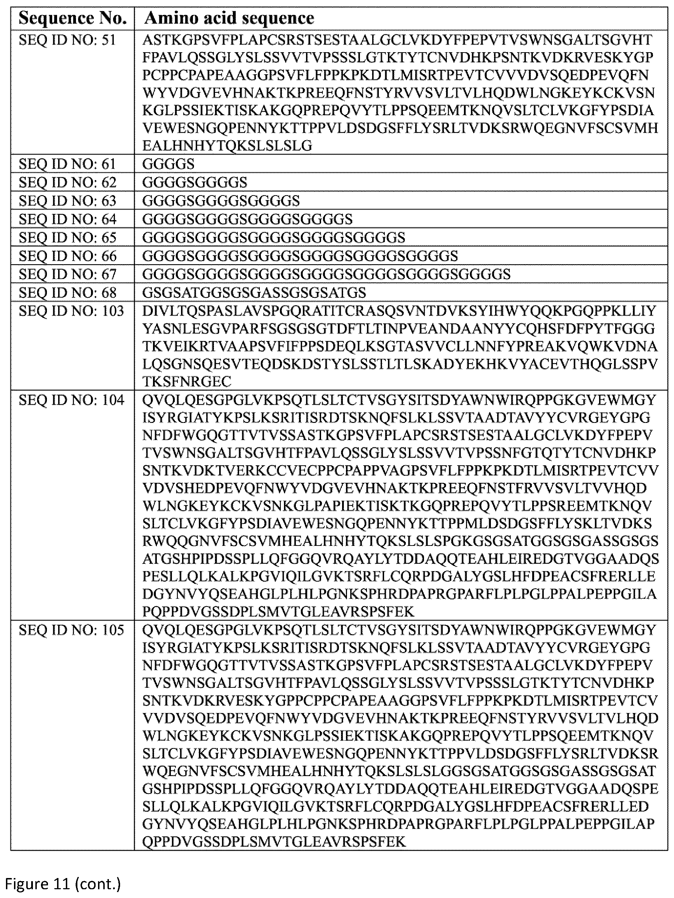

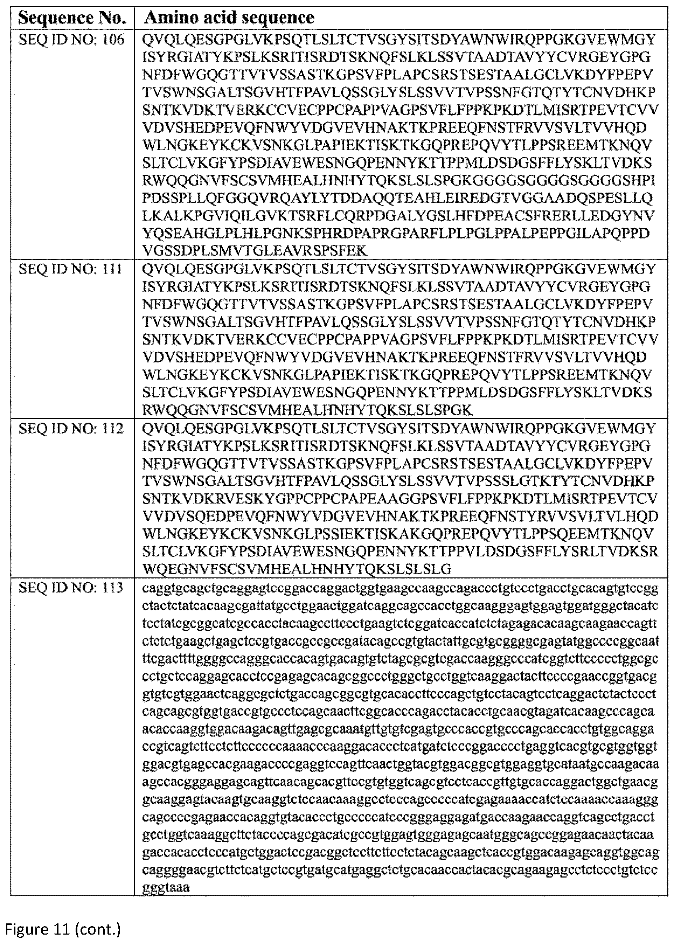

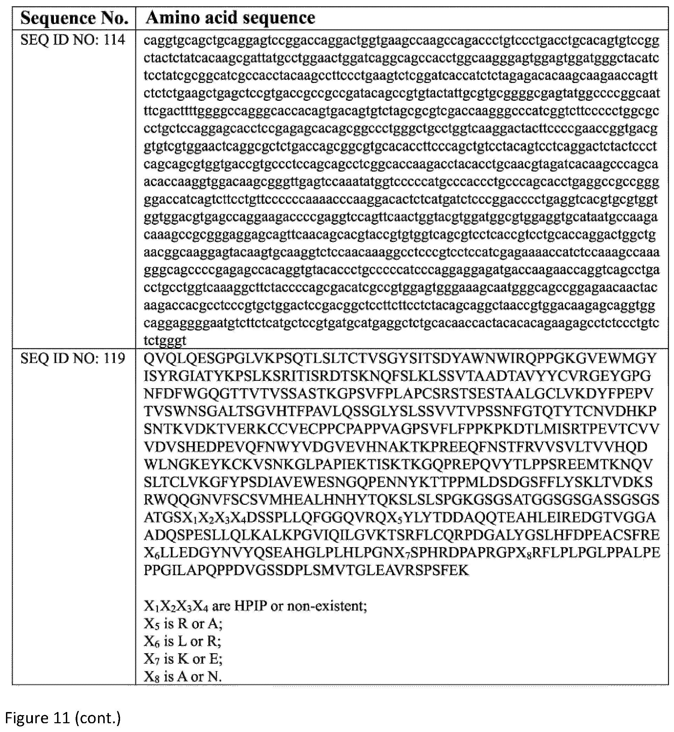

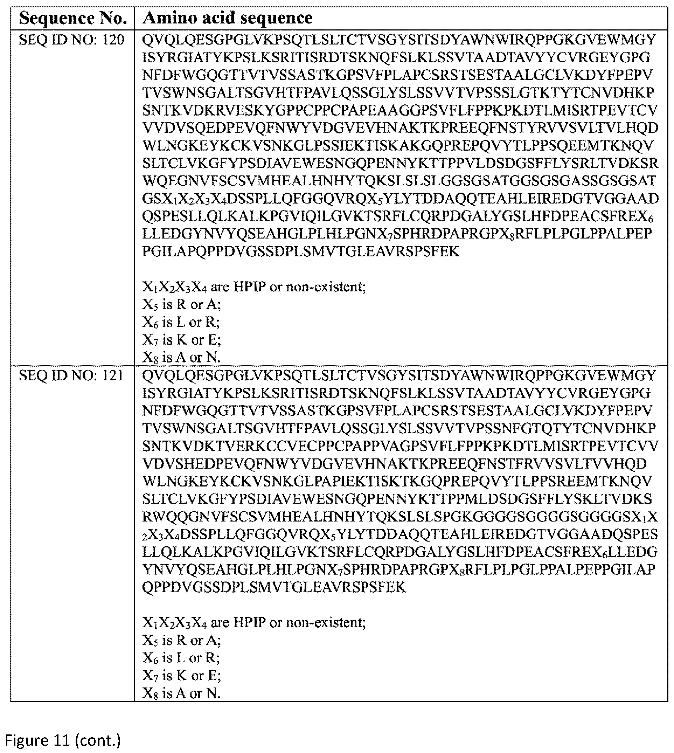

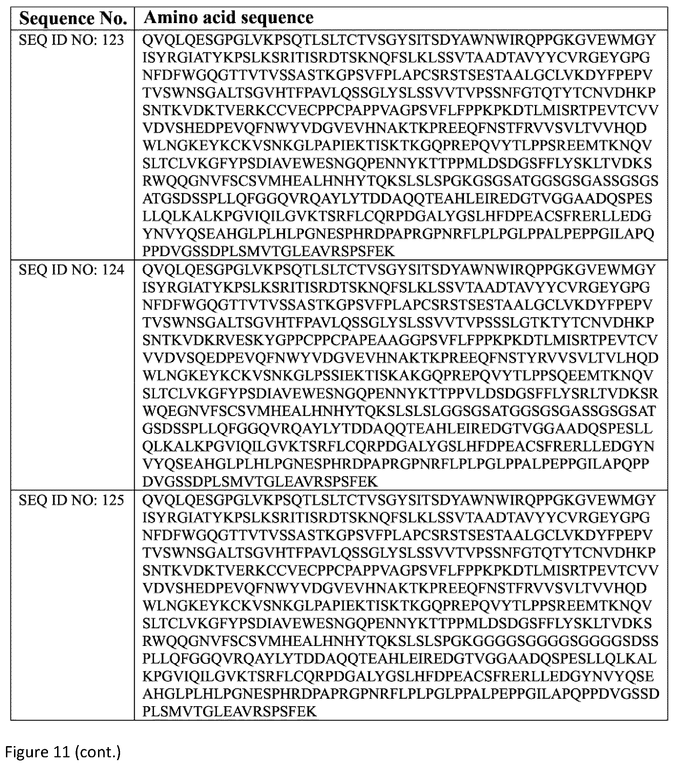

FIG. 11 shows the amino acid or nucleotide sequences as set forth in SEQ ID NO: 29 to SEQ ID NO: 51, SEQ ID NO: 61 to SEQ ID NO: 68, SEQ ID NO: 103 to SEQ ID NO: 106, SEQ ID NO: 111 to SEQ ID NO: 114, SEQ ID NO: 119 to SEQ ID NO: 121 and SEQ ID NO: 123 to SEQ ID NO: 125.

### Detailed Description of Embodiments

### Definitions

Unless defined otherwise herein, scientific and technical terms shall have the meanings understood by one of ordinary skill in the art. Generally, the nomenclature and techniques related to pharmacology, biology, biochemistry, cell and tissue culture, biology, molecular biology, immunology, microbiology, genetics and protein nucleic acid chemistry as well as hybridization as described herein are well known and commonly used in the art.

Standard one-letter or three-letter abbreviations are used herein to indicate polynucleotide and polypeptide sequences. The polypeptide sequence is written with the first amino acid residue (N') bearing an amino group at the leftmost position and the last amino acid residue (C') bearing a carboxy group at the rightmost position, for example the FGF21 fragment sequences involved herein: SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 115, SEQ ID NO: 116, SEQ ID NO: 117, SEQ ID NO: 118 and SEQ ID NO: 122. The 5' ends of the upstream chain of single-stranded and double-stranded nucleic acid sequences are on the left and the 3' ends thereof are on the right. The specific portion of a polypeptide can be represented by an amino acid residue number, such as amino acid 80 to amino acid 130, or represented by the actual residue of the site, such as Lys80 to Lys130. The specific polypeptide or polynucleotide sequence can also be described by explaining the difference thereof from the reference sequence.

The terms "peptide", "polypeptide" and "protein" each refer to a molecule comprising two or more amino acids that are interlinked by a peptide bond. These terms cover, for example, natural and artificial proteins, and polypeptide analogs of protein sequences (such as mutant proteins, variants and fusion proteins), and proteins that are post-transcriptionally or otherwise covalently or non-covalently modified. A peptide, polypeptide or protein can be monomeric or polymeric.

The term "polypeptide fragment" refers to a polypeptide that has an amino terminal and/or a carboxyl terminal deletion as compared to a corresponding full-length protein. A fragment may be, for example, at least 5, 6, 7, 8, 9, 10, 11, 12, 13, 14, 15, 20, 50, 70, 80, 90, 100, 150 or 200 amino acids in length. A fragment may be, for example, at most 1000, 750, 500, 250, 200, 175, 150, 125, 100, 90, 80, 70, 60, 50, 40, 30, 20, 15, 14, 13, 12, 11 or 10 amino acids in length. A fragment may further comprise, at either or both of its ends, one or more additional amino acids, for example, sequences of amino acids from different natural proteins (e.g., an Fc or a leucine zipper domain) or an artificial amino acid sequence (e.g., an artificial linker sequence).

Polypeptides herein include polypeptides that are modified in any way and for any reason, for example, to: (1) reduce susceptibility to proteolysis, (2) reduce susceptibility to oxidation, (3) alter the affinity for forming protein complexes, (4) alter the binding affinity, and (5) confer or modify other physiochemical or functional properties. Analogs comprise mutant proteins of polypeptides. For example, single or multiple amino acid substitutions (e.g., conservative amino acid substitutions) may be performed in natural sequences (e.g., in the portion of the polypeptide outside the domains that form intramolecular contacts). The "conservative amino acid substitution" is one that does not significantly alter the structural characteristics of a parent sequence (e.g., the substitution of amino acids shall not destroy a helix present in the parent sequence or interfere with other types of secondary structure that characterize the parent sequence or that are necessary for its functionality).

A "variant" of a polypeptide comprises an amino acid sequence in which one or more amino acid residues are inserted into, deleted from, and/or substituted into the amino acid sequence relative to another polypeptide sequence. Variants herein include variants of proteins, variants of antibodies, and variants of antibody fusion proteins. In embodiments herein, the FGF21 moiety comprised in the antibody fusion protein used has a variety of different variants, e.g., SEQ ID NO: 77 to SEQ ID NO: 89, SEQ ID NO: 115 to SEQ ID NO: 118 and SEQ ID NO: 122 describe such variant sequences of a wild-type FGF21 that retain comparable biological activity and protein structure to the wild-type FGF21, with insertions, deletions, and/or substitutions of one or more amino acid residues in the sequences compared to the wild-type FGF21. These FGF21 variants are fused to an antibody described herein below, thereby forming "variants" of a wild-type FGF21 fusion protein, e.g., SEQ ID NO: 104 to SEQ ID NO: 106, SEQ ID NO: 119 to SEQ ID NO: 121, SEQ ID NO: 123 to SEQ ID NO: 125 describe the heavy chain sequences of such variants of FGF21 fusion proteins.

A "derivative" of a polypeptide is a polypeptide that is chemically modified, e.g., by binding to another chemical moiety such as polyethylene glycol and albumin (such as human serum albumin), phosphorylation and glycosylation.

Unless otherwise stated, the term "antibody" includes antibodies comprising two full-length heavy chains and two full-length light chains, and derivatives, variants, fragments and mutant proteins thereof, and examples of which are listed below.

The term "antibody" is a protein comprising a moiety that binds to an antigen and, optionally, a scaffold or framework moiety that allows the antigen binding moiety to adopt a conformation that promotes the binding of the antibody to the antigen. Examples of antibodies include intact antibodies, antibody fragments (e.g., antigen-binding moieties of antibodies), antibody derivatives, antibody analogs, and antibody mutants, and their fusion proteins. The antibody may comprise, for example, an alternative protein scaffold or an artificial scaffold having grafted CDRs or CDR derivatives. The scaffold includes, but is not limited to an antibody-derived scaffold that is introduced, such as one that stabilizes the three-dimensional structure of the antibody, and a fully synthetic scaffold that contains, for example, a biocompatible polymer. See, e.g., Korndorfer et al., 2003, Proteins 53:121-129; Roque et al., 2004, Biotechnol. Prog. 20:639-654. In addition, the antibody can be a peptide antibody mimetic ("PAM") or a scaffold comprising an antibody mimetic using fibronectin as a scaffold.

An antibody may have, for example, the structure of a natural immunoglobulin. The "immunoglobulin" is a tetrameric molecule. In a natural immunoglobulin, each tetramer is composed of two identical pairs of polypeptide chains, each pair having one "light" chain (approximately 25 kDa) and one "heavy" chain (approximately 50-70 kDa). The amino terminal of each chain includes a variable domain of approximately 100 to 110 amino acids, which is primarily responsible for antigen recognition. The carboxyl terminal moiety of each chain defines a constant region which is primarily responsible for effector function. Human antibody light chains are classified as κ and λ light chains. Heavy chains are classified as µ, δ, α or ε heavy chains, and define the isotypes of the antigen as IgM, IgD, IgG, IgA and IgE, respectively. Within light and heavy chains, the variable and constant regions are linked by a "J" region of approximately 12 or more amino acids, with the heavy chain also including a "D" region of approximately 10 or more amino acids. See, Fundamental Immunology Ch.7 (Paul, ed., 2nd ed., Raven Press, 1989). The variable regions of each light/heavy chain pair form antibody binding sites, such that an intact immunoglobulin has two binding sites.

Natural immunoglobulin chains exhibit the same basic structure of relatively conservative framework regions (FRs) linked by three hypervariable regions, also referred to as complementarity determining regions or CDRs. From N terminus to C terminus, both light and heavy chains comprise domains FR1, CDR1, FR2, CDR2, FR3, CDR3 and FR4. The assignment of amino acids to each domain is in accordance with the definitions of Kabat et al., Sequences of Proteins of Immunological Interest, 5th edition, U.S. Dept. of Health and Human Services, PHS, NIH, NIH Publication No. 91-3242, 1991.

Unless otherwise specified, the "antibody" refers to an intact immunoglobulin or an antigen binding moiety thereof that may compete with an intact antibody for specific binding. Antigen binding moieties may be produced by recombinant DNA techniques or by enzymatic or chemical cleavage of intact antibodies. Antigen binding moieties include, in particular, Fab, Fab', F(ab')₂, Fv, domain antibodies (dAbs), fragments including complementarity determining regions (CDRs), single-chain antibodies (scFv), chimeric antibodies, diabodies, triabodies, tetrabodies, and polypeptides that comprise at least a portion of an immunoglobulin that is sufficient to confer specific antigen binding to the polypeptides.

An Fab fragment is a monovalent fragment having V_{L}, V_{H}, C_{L} and C_{H1} domains; an F(ab')₂ fragment is a divalent fragment having two Fab fragments linked by a disulfide bond at the hinge region; an Fv fragment has V_{H} and V_{L} domains; and a dAb fragment has a V_{H} domain, a V_{L} domain, or an antigen binding fragment of a V_{H} or V_{L} domain (U.S. Pat. No. US 6,846,634 and US 6,696,245; U.S. Patent Application Publication No. US 2005/0202512, US 2004/0202995, US 2004/0038291, US 2004/0009507 and US 2003/0039958; Ward et al., 1989, Nature 341:544-546).

A single-chain antibody (scFv) is a fusion protein in which V_{L} and V_{H} regions are linked by a linker (for example, a synthetic sequence of amino acid residues) to form a continuous protein, wherein the linker is long enough to allow the protein chain to fold back on itself and form a monovalent antigen binding site (see, for example, Bird et al., 1988, Science 242:423-26; and Huston et al., 1988, Proc. Natl. Acad. Sci. U.S.A. 85:5879-83).

A diabody is a divalent antibody comprising two polypeptide chains, wherein each polypeptide chain comprises V_{H} and V_{L} domains linked by a linker that is too short to allow for pairing of the two domains on the same chain, thus allowing each domain to pair with a complementary domain on another polypeptide chain (see, for example, Holliger et al., 1993, Proc. Natl. Acad. Sci. U.S.A. 90:6444-48; and Poljak et al., 1994, Structure 2:1121-23). If the two polypeptide chains of a diabody are identical, then a diabody resulting from their pairing will have identical antigen binding sites. Polypeptide chains having different sequences can be used to prepare a diabody having different antigen binding sites. Similarly, triabodies and tetrabodies are antibodies that comprise three and four polypeptide chains, respectively, and form three and four antigen binding sites, respectively, which may be identical or different.

Complementarity determining regions (CDRs) and framework regions (FRs) of a given antibody may be identified herein using the method described in Kabat et al., Sequences of Proteins of Immunological Interest, 5th edition, U.S. Dept. of Health and Human Services, PHS, NIH, NIH Publication No. 91-3242, 1991. One or more CDRs may be incorporated into a molecule either covalently or noncovalently to make it an antibody. An antibody may incorporate CDR(s) as part of a larger polypeptide chain, may covalently link CDR(s) to another polypeptide chain, or may incorporate CDR(s) noncovalently. CDRs allow for specific binding of an antibody to a specific associated antigen.

Antibodies may have one or more binding sites. If there is more than one binding site, the binding site may be identical to or different from one another. For example, a natural human immunoglobulin usually has two identical binding sites, while a "bispecific" or "bifunctional" antibody has two different binding sites.

The term "murine-derived antibody" includes antibodies that have one or more variable and constant regions derived from mouse immunoglobulin sequences.

The term "humanized antibody" is an antibody made by grafting the complementarity determining region sequences of a mouse antibody molecule into the framework of human antibody variable regions.

The term "antigen binding domain", "antigen binding region" or "antigen binding site" is a portion of an antibody that comprises amino acid residues that interact with an antigen and contributes to the specificity and affinity of the antibody to the antigen. For an antibody that specifically binds to an antigen thereof, this will include at least portion of at least one of its CDR domains.

The term "epitope" is a portion of a molecule that is bound by an antibody (for example, by an antibody). An epitope may comprise non-contiguous portions of a molecule (for example, in a polypeptide, amino acid residues that are not contiguous in the primary sequence of the polypeptide but that, in the tertiary and quaternary structures of the polypeptide, are near enough to be bound by an antibody).

The "percent identity" of two polynucleotide or two polypeptide sequences is determined by comparing the sequences using the GAP computer program (a part of the GCG Wisconsin Package, version 10.3 (Accelrys, San Diego, CA)) using its default parameters.

The terms "polynucleotide", "oligonucleotide" and "nucleic acid" are used interchangeably throughout the full text and include DNA molecules (e.g., cDNA or genomic DNA), RNA molecules (e.g., mRNA), DNA or RNA analogs and hybrids thereof produced using nucleotide analogs (e.g., peptide nucleic acids and non-natural nucleotide analogs). Nucleic acid molecules may be single- or double-stranded. In one embodiment, the nucleic acid molecule herein comprises a contiguous open reading frame encoding the antibody or the fragment, derivative, mutant protein or variant thereof provided herein.

If the sequences of two single-stranded polynucleotides are "complementary" to each other, the two single-stranded polynucleotides can be aligned in an anti-parallel orientation, so that every nucleotide in one polynucleotide is opposite to its complementary nucleotide in another polynucleotide, without the introduction of gaps, and without unpaired nucleotides at the 5' or 3' end of either sequence. If the two polynucleotides can hybridize to each other under moderately stringent conditions, one polynucleotide is "complementary" to another polynucleotide. Thus, one polynucleotide may be complementary to another polynucleotide, but not the complementary sequence thereof.

The term "vector" is a nucleic acid that can be used to introduce another nucleic acid ligated thereto into a cell. One type of vector is a "plasmid", which refers to a linear or circular double-stranded DNA molecule to which additional nucleic acid segments can be ligated. Another type of vector is a viral vector (e.g., replication-defective retroviruses, adenoviruses and adenovirus-associated viruses) in which additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell when introduced into the host cell, and thus are replicated along with the host genome. The "expression vector" is a type of vector that can direct the expression of a selected polynucleotide.

If a regulatory sequence affects the expression (e.g., the level, timing, or location of expression) of a nucleotide sequence, the nucleotide sequence is "operably linked" to the regulatory sequence. The "regulatory sequence" is a nucleic acid that affects the expression (e.g., the level, timing, or location of expression) of a nucleic acid to which it is operably linked. A regulatory gene, for example, act directly on a regulated nucleic acid or through one or more other molecules (e.g., polynucleotides that bind to a regulatory sequence and/or nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology, Volume 185, Academic Press, San Diego, CA; and Baron et al., 1995, Nucleic Acids Res. 23:3605-06.

The term "host cell" is a cell that can be used to express a nucleic acid such as the nucleic acid provided herein. The host cell may be a prokaryote, for example, E. coli, or it can be eukaryote, such as a unicellular eukaryote (e.g., a yeast or other fungi), a plant cell (e.g., a tobacco or tomato plant cell), an animal cell (e.g., a human cell, a monkey cell, a hamster cell, a rat cell, a mouse cell or an insect cell) or a hybridoma. Generally, a host cell is a culture cell that can be transformed or transfected with a polypeptide-encoding nucleic acid, which can then be expressed in the host cell. The phrase "recombinant host cell" can be used to denote a host cell that is transformed or transfected with a nucleic acid to be expressed. A host cell may also be a cell that comprises the nucleic acid but does not express it at a desired level, unless a regulatory sequence is introduced into the host cell so that it is operably linked to the nucleic acid. It should be understood that the term "host cell" refers not only to the specific subject cell but also to the progeny or possible progeny of such a cell. Because certain modifications may occur in succeeding generations due to, e.g., mutations or environmental influence, such progeny may not, in fact, be identical to the parent cell, but still fall within the scope of the term as used herein.

### Gastric inhibitory polypeptide receptor

The gastric inhibitory polypeptide receptor belongs to type B of the family of seven-transmembrane G protein-coupled receptors, which is coupled to one or more intracellular signaling pathways through a heterotrimeric guanine nucleotide binding protein (G protein) (Drucker et al., 2006, Cell Metab. 3:153-65). Studies to date have found that the GIPR is mainly expressed on the surface of pancreatic islet beta cells and adipocytes (Ravn et al., 2013, J. Biol. Chem. 288:19760-72), is involved in both glucose and lipid metabolism processes in humans and is therefore also closely associated with diabetes, obesity and related conditions (Skaw et al., 2016, Diabetes Obes. Metab. 18:847-854). As used herein, both of "a human GIPR" and "an hGIPR" refer to the human-derived gastric inhibitory polypeptide receptor and may be used interchangeably. As used herein, both of "a murine GIPR" and "an mGIPR" refer to the murine-derived gastric inhibitory polypeptide receptor, and may also be used interchangeably.

In one embodiment, the antibody provided herein is an antibody that specifically binds to a human GIPR. In another embodiment, the antibody provided herein is an antibody that specifically binds to a GIPR on cell membranes, and the antibody can inhibit or block the transduction of GIP signals within these cells. In another embodiment, the antibody provided herein is an antibody that specifically binds to a human GIPR, and the antibody can bind to a GIPR of other species (e.g., monkeys or mice) and block GIP signaling in these species. In a further embodiment, the antibody provided herein is a murine-derived antibody that binds to a human GIPR, and the antibody can bind to a GIPR of other species (e.g., monkeys).

In one embodiment, the amino acid and polynucleotide sequences of a GIPR are listed below, and the data of the sequences are derived from the GeneBank database of the National Center for Biotechnology Information in the United States and the Uniprot database of the European Bioinformatics Institute:
Homo sapiens polynucleotide, Accession No.: S79852;
Homo sapiens amino acid, Accession No.: AAB35419.2.
Gastric inhibitory polypeptide receptor antibody (GIPR antibody)

In one embodiment, provided herein is a GIPR antibody. In another embodiment, the GIPR antibody provided herein is an intact GIPR antibody. In another embodiment, the GIPR antibody provided herein is a GIPR antibody fragment. In another embodiment, the GIPR antibody provided herein is a GIPR antibody derivative. In another embodiment, the GIPR antibody provided herein is a GIPR antibody mutein. In a further embodiment, the GIPR antibody provided herein is a GIPR antibody variant.

In one embodiment, the GIPR antibody provided herein comprises one, two, three, four, five, or six amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11;
b. light chain CDR2 amino acid sequences: YAS, SEQ ID NO: 2;
c. light chain CDR3 amino acid sequences: SEQ ID NO: 3, SEQ ID NO: 4;
d. heavy chain CDR1 amino acid sequence: SEQ ID NO: 12;
e. heavy chain CDR2 amino acid sequence: SEQ ID NO: 13; and
f. heavy chain CDR3 amino acid sequence: SEQ ID NO: 14.

Table 1 lists the light chain CDR amino acid sequences of the GIPR antibody provided herein, and the corresponding polynucleotide coding sequences thereof. Table 2 lists the heavy chain CDR amino acid sequences of the GIPR antibody provided herein, and the corresponding polynucleotide coding sequences thereof.

**Table 1: Light chain CDR amino acid sequences and polynucleotide coding sequences thereof**

| | **CDR1** | **CDR2** | **CDR3** |
|---|---|---|---|
| A-1 nucleotide | cagtccgtgaacacagatgtgaagtcttat (SEQ ID NO: 15) | tatgccagc or tatgccagcaac (SEQ ID NO: 16) | cagcacagcttcgatttcccctacacc (SEQ ID NO: 17) |
| A-1 amino acid | QSVNTDVKSY (SEQ ID NO: 1) | YAS or YASN (SEQ ID NO: 2) | QHSFDFPYT (SEQ ID NO: 3) |
| A-2 nucleotide | cagtccgtgaacacagacgtgtactcttat (SEQ ID NO: 19) | tatgccagc or tatgccagcaac (SEQ ID NO: 16) | cagcacagcttcgatttcccctacacc (SEQ ID NO: 17) |
| A-2 amino acid | QSVNTDVYSY (SEQ ID NO: 5) | YAS or YASN (SEQ ID NO: 2) | QHSFDFPYT (SEQ ID NO: 3) |
| A-3 nucleotide | cagtccgtgaacacagccgactactcttat (SEQ ID NO: 20) | tatgccagc or tatgccagcaac (SEQ ID NO: 16) | cagcacagcttcgatttcccctacacc (SEQ ID NO: 17) |
| A-3 amino acid | QSVNTADYSY (SEQ ID NO: 6) | YAS or YASN (SEQ ID NO: 2) | QHSFDFPYT (SEQ ID NO: 3) |
| A-4 nucleotide | cagtccgtgaacacaaaggactactcttat (SEQ ID NO: 21) | tatgccagc or tatgccagcaac (SEQ ID NO: 16) | cagcacagcttcgatttcccctacacc (SEQ ID NO: 17) |
| A-4 amino acid | QSVNTKDYSY (SEQ ID NO: 7) | YAS or YASN (SEQ ID NO: 2) | QHSFDFPYT (SEQ ID NO: 3) |
| A-5 nucleotide | cagtccgtgaacacagatgtgggctcttat (SEQ ID NO: 22) | tatgccagc or tatgccagcaac (SEQ ID NO: 16) | cagcacagcttcgatttcccctacaccg (SEQ ID NO: 17) |
| A-5 amino acid | QSVNTDVGSY (SEQ ID NO: 8) | YAS or YASN (SEQ ID NO: 2) | QHSFDFPYT (SEQ ID NO: 3) |
| A-6 nucleotide | cagtccgtgaacacagacagctactcttat (SEQ ID NO: 23) | tatgccagc or tatgccagcaac (SEQ ID NO: 16) | cagcacagcttcgatttcccctacacc (SEQ ID NO: 17) |
| A-6 amino acid | QSVNTDSYSY (SEQ ID NO: 9) | YAS or YASN (SEQ ID NO: 2) | QHSFDFPYT (SEQ ID NO: 3) |
| A-7 nucleotide | cagtccgtgaacacagatgtgaagtcttat (SEQ ID NO: 24) | tatgccagc or tatgccagcaac (SEQ ID NO: 16) | cagcacagcttcgatttcccctacacc (SEQ ID NO: 17) |
| A-7 amino acid | QSVNTDVKSY (SEQ ID NO: 10) | YAS or YASN (SEQ ID NO: 2) | QHSFDFPYT (SEQ ID NO: 3) |
| A-8 nucleotide | cagtccgtgaacaaggatgtgtactctta (SEQ ID NO: 25) | tatgccagc or tatgccagcaac (SEQ ID NO: 16) | cagcacagcttcgatttcccctacacc (SEQ ID NO: 17) |
| A-8 amino acid | QSVNKDVYSY (SEQ ID NO: 11) | YAS or YASN (SEQ ID NO: 2) | QHSFDFPYT (SEQ ID NO: 3) |
| A-9 nucleotide | cagtccgtgaacacagatgtgaagtcttat (SEQ ID NO: 15) | tatgccagc or tatgccagcaac (SEQ ID NO: 16) | cagcacagcttcgatgacccctac (SEQ ID NO: 18) |
| A-9 amino acid | QSVNTDVKSY (SEQ ID NO: 1) | YAS or YASN (SEQ ID NO: 2) | QHSFDDPYT (SEQ ID NO: 4) |

**Table 2: Heavy chain CDR amino acid sequences and polynucleotide coding sequences thereof**

| | **CDR1** | **CDR2** | **CDR3** |
|---|---|---|---|
| A-1 nucleotide | ggctactctatcacaagcgattatgcc (SEQ ID NO: 26) | atctcctatcgcggcatcgcc (SEQ ID NO: 27) | gtgcggggcgagtatggccccgg caatttcgacttt (SEQ ID NO: 28) |
| A-1 amino acid | GYSITSDYA (SEQ ID NO: 12) | ISYRGIA (SEQ ID NO: 13) | VRGEYGPGNFDF (SEQ ID NO: 14) |

In one embodiment, the antibody provided herein comprises a sequence that differs from one of the CDR amino acid sequences listed in Tables 1 and 2 by 5, 4, 3, 2 or 1 single amino acid addition, substitution and/or deletion. In another embodiment, the antibody provided herein comprises a sequence that differs from one of the CDR amino acid sequences listed in Tables 1 and 2 by 4, 3, 2 or 1 single amino acid addition, substitution and/or deletion.

In another embodiment, the antibody provided herein comprises a sequence that differs from one of the CDR amino acid sequences listed in Tables 1 and 2 by 3, 2 or 1 single amino acid addition, substitution and/or deletion.

In another embodiment, the antibody provided herein comprises a sequence that differs from one of the CDR amino acid sequences listed in Tables 1 and 2 by 2 or 1 single amino acid addition, substitution and/or deletion.

In a further embodiment, the antibody provided herein comprises a sequence that differs from one of the CDR amino acid sequences listed in Tables 1 and 2 by 1 single amino acid addition, substitution and/or deletion.

In one embodiment, the GIPR antibody provided herein comprises one or two amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11; and
b. heavy chain CDR1 amino acid sequence: SEQ ID NO: 12.

In another embodiment, the GIPR antibody provided herein comprises one or two amino acid sequences, wherein each amino acid sequence is independently selected from the amino acid sequences listed below:
a. light chain CDR2 amino acid sequences: YAS, SEQ ID NO: 2 and
b. heavy chain CDR2 amino acid sequence: SEQ ID NO: 13.

In another embodiment, the GIPR antibody provided herein comprises one or two amino acid sequences, wherein each amino acid sequence is independently selected from the amino acid sequences listed below:
a. light chain CDR3 amino acid sequences: SEQ ID NO: 3, SEQ ID NO: 4; and
b. heavy chain CDR3 amino acid sequence: SEQ ID NO: 14.

In another embodiment, the GIPR antibody provided herein comprises one, two, three, or four amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11;
b. heavy chain CDR1 amino acid sequence: SEQ ID NO: 12;
c. light chain CDR2 amino acid sequences: YAS, SEQ ID NO: 2; and
d. heavy chain CDR2 amino acid sequence: SEQ ID NO: 13.

In another embodiment, the GIPR antibody provided herein comprises one, two, three, or four amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11;
b. heavy chain CDR1 amino acid sequence: SEQ ID NO: 12;
c. light chain CDR3 amino acid sequences: SEQ ID NO: 3, SEQ ID NO: 4; and
d. heavy chain CDR3 amino acid sequence: SEQ ID NO: 14.

In a further embodiment, the GIPR antibody provided herein comprises one, two, three, or four amino acid sequences, wherein each amino acid sequence is independently selected from the amino acid sequences listed below:
a. light chain CDR2 amino acid sequences: YAS, SEQ ID NO: 2;
b. heavy chain CDR2 amino acid sequence: SEQ ID NO: 13;
c. light chain CDR3 amino acid sequences: SEQ ID NO: 3, SEQ ID NO: 4; and
d. heavy chain CDR3 amino acid sequence: SEQ ID NO: 14.

In one embodiment, the GIPR antibody provided herein comprises one, two, or three amino acid sequences, wherein each amino acid sequence is independently selected from the amino acid sequences listed below: SEQ ID NO: 1, YAS, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

In another embodiment, the GIPR antibody provided herein comprises one, two, or three amino acid sequences, wherein each amino acid sequence is independently selected from the amino acid sequences listed below: SEQ ID NO: 12, SEQ ID NO: 13, SEQ ID NO: 14.

In one embodiment, the GIPR antibody provided herein comprises a combination of light and heavy chain CDR1 amino acid sequences independently selected from the list below: SEQ ID NO: 1 and SEQ ID NO: 12, SEQ ID NO: 5 and SEQ ID NO: 12, SEQ ID NO: 6 and SEQ ID NO: 12, SEQ ID NO: 7 and SEQ ID NO: 12, SEQ ID NO: 8 and SEQ ID NO: 12, SEQ ID NO: 9 and SEQ ID NO: 12, SEQ ID NO: 10 and SEQ ID NO: 12, and SEQ ID NO: 11 and SEQ ID NO: 12.

In another embodiment, the GIPR antibody provided herein comprises a combination of light and heavy chain CDR2 amino acid sequences independently selected from the list below: YAS and SEQ ID NO: 13, and SEQ ID NO: 2 and SEQ ID NO: 13.

In a further embodiment, the GIPR antibody provided herein comprise a combination of light and heavy chain CDR3 amino acid sequences independently selected from the list below: SEQ ID NO: 3 and SEQ ID NO: 14, SEQ ID NO: 4 and SEQ ID NO: 14.

In one embodiment, the GIPR antibody provided herein comprises:
a. a combination of light and heavy chain CDR1 amino acid sequences independently selected from the list below: SEQ ID NO: 1 and SEQ ID NO: 12, SEQ ID NO: 5 and SEQ ID NO: 12, SEQ ID NO: 6 and SEQ ID NO: 12, SEQ ID NO: 7 and SEQ ID NO: 12, SEQ ID NO: 8 and SEQ ID NO: 12, SEQ ID NO: 9 and SEQ ID NO: 12, SEQ ID NO: 10 and SEQ ID NO: 12, and SEQ ID NO: 11 and SEQ ID NO: 12; and
b. a combination of light and heavy chain CDR2 amino acid sequences independently selected from the list below: YAS and SEQ ID NO: 13, and SEQ ID NO: 2 and SEQ ID NO: 13.

In another embodiment, the GIPR antibody provided herein comprises:
a. a combination of light and heavy chain CDR1 amino acid sequences independently selected from the list below: SEQ ID NO: 1 and SEQ ID NO: 12, SEQ ID NO: 5 and SEQ ID NO: 12, SEQ ID NO: 6 and SEQ ID NO: 12, SEQ ID NO: 7 and SEQ ID NO: 12, SEQ ID NO: 8 and SEQ ID NO: 12, SEQ ID NO: 9 and SEQ ID NO: 12, SEQ ID NO: 10 and SEQ ID NO: 12, and SEQ ID NO: 11 and SEQ ID NO: 12; and
b. a combination of light and heavy chain CDR3 amino acid sequences independently selected from the list below: SEQ ID NO: 3 and SEQ ID NO: 14, SEQ ID NO: 4 and SEQ ID NO: 14.

In another embodiment, the GIPR antibody provided herein comprises:
a. a combination of light and heavy chain CDR2 amino acid sequences independently selected from the list below: YAS and SEQ ID NO: 13, and SEQ ID NO: 2 and SEQ ID NO: 13; and
b. a combination of light and heavy chain CDR3 amino acid sequences independently selected from the list below: SEQ ID NO: 3 and SEQ ID NO: 14, SEQ ID NO: 4 and SEQ ID NO: 14.

In a further embodiment, the GIPR antibody provided herein comprises:
a. a combination of light and heavy chain CDR1 amino acid sequences independently selected from the list below: SEQ ID NO: 1 and SEQ ID NO: 12, SEQ ID NO: 5 and SEQ ID NO: 12, SEQ ID NO: 6 and SEQ ID NO: 12, SEQ ID NO: 7 and SEQ ID NO: 12, SEQ ID NO: 8 and SEQ ID NO: 12, SEQ ID NO: 9 and SEQ ID NO: 12, SEQ ID NO: 10 and SEQ ID NO: 12, and SEQ ID NO: 11 and SEQ ID NO: 12;
b. a combination of light and heavy chain CDR2 amino acid sequences independently selected from the list below: YAS and SEQ ID NO: 13, and SEQ ID NO: 2 and SEQ ID NO: 13; and
c. a combination of light and heavy chain CDR3 amino acid sequences independently selected from the list below: SEQ ID NO: 3 and SEQ ID NO: 14, SEQ ID NO: 4 and SEQ ID NO: 14.

In one embodiment, the GIPR antibody provided herein comprises:
a. a combination of light and heavy chain CDR1, CDR2, and CDR3 amino acid sequences: SEQ ID NO: 1, YAS or SEQ ID NO: 2, SEQ ID NO: 3, SEQ ID NO: 12, SEQ ID NO: 13, and SEQ ID NO: 14;
b. a combination of light and heavy chain CDR1, CDR2, and CDR3 amino acid sequences: SEQ ID NO: 5, YAS or SEQ ID NO: 2, SEQ ID NO: 3, SEQ ID NO: 12, SEQ ID NO: 13, and SEQ ID NO: 14;
c. a combination of light and heavy chain CDR1, CDR2, and CDR3 amino acid sequences: SEQ ID NO: 6, YAS or SEQ ID NO: 2, SEQ ID NO: 3, SEQ ID NO: 12, SEQ ID NO: 13, and SEQ ID NO: 14;
d. a combination of light and heavy chain CDR1, CDR2, and CDR3 amino acid sequences: SEQ ID NO: 7, YAS or SEQ ID NO: 2, SEQ ID NO: 3, SEQ ID NO: 12, SEQ ID NO: 13, and SEQ ID NO: 14;
e. a combination of light and heavy chain CDR1, CDR2, and CDR3 amino acid sequences: SEQ ID NO: 8, YAS or SEQ ID NO: 2, SEQ ID NO: 3, SEQ ID NO: 12, SEQ ID NO: 13, and SEQ ID NO: 14;
f. a combination of light and heavy chain CDR1, CDR2, and CDR3 amino acid sequences: SEQ ID NO: 9, YAS or SEQ ID NO: 2, SEQ ID NO: 3, SEQ ID NO: 12, SEQ ID NO: 13, and SEQ ID NO: 14;
g. a combination of light and heavy chain CDR1, CDR2, and CDR3 amino acid sequences: SEQ ID NO: 10, YAS or SEQ ID NO: 2, SEQ ID NO: 3, SEQ ID NO: 12, SEQ ID NO: 13, and SEQ ID NO: 14; or
h. a combination of light and heavy chain CDR1, CDR2, and CDR3 amino acid sequences: SEQ ID NO: 11, YAS or SEQ ID NO: 2, SEQ ID NO: 3, SEQ ID NO: 12, SEQ ID NO: 13, and SEQ ID NO: 14.
i. a combination of light and heavy chain CDR1, CDR2, and CDR3 amino acid sequences: SEQ ID NO: 1, YAS or SEQ ID NO: 2, SEQ ID NO: 4, SEQ ID NO: 12, SEQ ID NO: 13, and SEQ ID NO: 14.

In one embodiment, the GIPR antibody provided herein comprises one or two amino acid sequences, wherein each amino acid sequence is independently selected from the amino acid sequences listed below:
a. light chain variable domain amino acid sequences: SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, and SEQ ID NO: 37; and an amino acid sequence that is at least 80%, at least 85%, at least 90%, or at least 95% identical to any of these sequences; and
b. heavy chain variable domain amino acid sequence: SEQ ID NO: 38; and an amino acid sequence that is at least 80%, at least 85%, at least 90%, or at least 95% identical thereto.

In another embodiment, the polynucleotide coding sequence of the GIPR antibody provided herein comprises one or two polynucleotide coding sequences, wherein each polynucleotide coding sequence is independently selected from the polynucleotide sequences listed below:
a. light chain variable domain polynucleotide coding sequences: SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 and SEQ ID NO: 47; and a polynucleotide coding sequence that is at least 80%, at least 85%, at least 90%, or at least 95% identical to any of these sequences; and
b. heavy chain variable domain polynucleotide coding sequence: SEQ ID NO: 48; and a polynucleotide coding sequence that is at least 80%, at least 85%, at least 90%, or at least 95% identical thereto.

In one embodiment, the GIPR antibody provided herein comprises an amino acid sequence independently selected from the list below: SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, and SEQ ID NO: 37.

In another embodiment, the GIPR antibody provided herein comprises an amino acid sequence independently selected from the list below: SEQ ID NO: 38.

In one embodiment, the GIPR antibody provided herein comprises a combination of light and heavy chain variable domain amino acid sequences independently selected from the list below: SEQ ID NO: 29 and SEQ ID NO: 38, SEQ ID NO: 30 and SEQ ID NO: 38, SEQ ID NO: 31 and SEQ ID NO: 38, SEQ ID NO: 32 and SEQ ID NO: 38, SEQ ID NO: 33 and SEQ ID NO: 38, SEQ ID NO: 34 and SEQ ID NO: 38, SEQ ID NO: 35 and SEQ ID NO: 38, SEQ ID NO: 36 and SEQ ID NO: 38, and SEQ ID NO: 37 and SEQ ID NO: 38.

In one embodiment, the GIPR antibody provided herein comprises an amino acid sequence independently selected from the list below: SEQ ID NO: 29, SEQ ID NO: 30, SEQ ID NO: 34, SEQ ID NO: 35, SEQ ID NO: 36 and SEQ ID NO: 37.

In another embodiment, the GIPR antibody provided herein comprises a combination of light and heavy chain variable domain amino acid sequences independently selected from the list below: SEQ ID NO: 29 and SEQ ID NO: 38 (L1H1), SEQ ID NO: 30 and SEQ ID NO: 38 (L2H1), SEQ ID NO: 31 and SEQ ID NO: 38 (L3H1), SEQ ID NO: 32 and SEQ ID NO: 38 (L4H1), SEQ ID NO: 33 and SEQ ID NO: 38 (L5H1), SEQ ID NO: 34 and SEQ ID NO: 38 (L6H1), SEQ ID NO: 35 and SEQ ID NO: 38 (L7H1), SEQ ID NO: 36 and SEQ ID NO: 38 (L8H1), and SEQ ID NO: 37 and SEQ ID NO: 38 (L9H1).

The GIPR antibody provided herein may also be represented herein by the "LxHy" notation, where "x" corresponds to a code number of a light chain variable region sequence; and "y" corresponds to a code number of a heavy chain variable region sequence. For example, L2H2 refers to an intact antibody having a light chain variable region comprising the amino acid sequence SEQ ID NO: 30 (L2) and a heavy chain variable region comprising the amino acid sequence SEQ ID NO: 38 (H1).

In one embodiment, the GIPR antibody provided herein comprises one or two amino acid sequences, wherein each amino acid sequence is independently selected from the amino acid sequences listed below:
a. light chain constant region amino acid sequence: SEQ ID NO: 49; and
b. heavy chain constant region amino acid sequences: SEQ ID NO: 50, SEQ ID NO: 51.

In one embodiment, the GIPR antibody provided herein comprises one or two amino acid sequences, wherein each amino acid sequence is independently selected from combinations of light and heavy chain constant region amino acid sequences listed below: SEQ ID NO: 49 and SEQ ID NO: 50, SEQ ID NO: 49 and SEQ ID NO: 51.

In one embodiment, the GIPR antibody provided herein comprises the amino acid sequences of the light and heavy chain CDRs listed herein and FRs (frameworks). The amino acid sequences of FRs are contained in the light or heavy chain variable domain amino acid sequences and are not separately displayed. In one embodiment, the antibody comprises a light chain CDR1 sequence listed herein. In another embodiment, the antibody comprises a light chain CDR2 sequence listed herein. In another embodiment, the antibody comprises a light chain CDR3 sequence listed herein. In another embodiment, the antibody comprises a heavy chain CDR1 sequence listed herein. In another embodiment, the antibody comprises a heavy chain CDR2 sequence listed herein. In another embodiment, the antibody comprises a heavy chain CDR3 sequence listed herein. In another embodiment, the antibody comprises a light chain FR1 sequence herein. In another embodiment, the antibody comprises a light chain FR2 sequence herein. In another embodiment, the antibody comprises a light chain FR3 sequence herein. In another embodiment, the antibody comprises a light chain FR4 sequence herein. In another embodiment, the antibody comprises a heavy chain FR1 sequence herein. In another embodiment, the antibody comprises a heavy chain FR2 sequence herein. In another embodiment, the antibody comprises a heavy chain FR3 sequence herein. In a further embodiment, the antibody comprises a heavy chain FR4 sequence herein.

In one embodiment, the light chain CDR3 sequence of the antibody differs from one of the light chain CDR3 amino acid sequences SEQ ID NO: 3 and SEQ ID NO: 4 listed herein by no more than 6, 5, 4, 3, 2 or 1 single amino acid addition, substitution and/or deletion. In another embodiment, the antibody further comprises 1, 2, 3, 4, 5 or 6 combinations of the light and heavy chain CDR sequences listed herein.

In one embodiment, the GIPR antibody provided herein comprises a light chain variable domain amino acid sequence selected from L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37) light chain variable domain sequences listed herein. In one embodiment, the light chain variable domain amino acid sequence of the GIPR antibody differs from one of the light chain variable domain amino acid sequences L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37) by 15, 14, 13, 12, 11, 10, 9, 8, 7, 6, 5, 4, 3, 2, or 1 amino acid, wherein the difference in each sequence is independently a deletion, insertion or substitution of an amino acid residue. In another embodiment, the light chain variable domain amino acid sequence of the GIPR antibody is at least 70%, at least 75%, at least 80%, at least 85%, at least 90%, at least 95%, at least 97%, or at least 99% identical to one of the light chain variable domain amino acid sequences L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37). In another embodiment, the light chain variable domain polynucleotide coding sequence of the GIPR antibody is at least 70%, at least 75%, at least 80%, at least 85%, at least 90%, at least 95%, at least 97%, or at least 99% identical to the polynucleotide coding sequence of one of L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37). In another embodiment, the light chain variable domain polynucleotide coding sequence of the GIPR antibody comprises a polynucleotide sequence that hybridizes to the complementary sequence of the polynucleotide coding sequence of one of light chain variable domains L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37) under moderately stringent conditions. In a further embodiment, the light chain variable domain polynucleotide coding sequence of the GIPR antibody comprises a polynucleotide sequence that hybridizes to the complementary sequence of the polynucleotide coding sequence of one of light chain variable domains L1 (SEQ ID NO: 29), L2 (SEQ ID NO: 30), L6 (SEQ ID NO: 34), L7 (SEQ ID NO: 35), L8 (SEQ ID NO: 36), and L9 (SEQ ID NO: 37) under stringent conditions.

In one embodiment, the GIPR antibody provided herein comprises a heavy chain variable domain amino acid sequence selected from the heavy chain variable domain sequence H1 (SEQ ID NO: 38) listed herein.

In one embodiment, the antibody provided herein is an antibody comprising a combination of SEQ ID NO: 29 and SEQ ID NO: 38 (L1H1), SEQ ID NO: 30 and SEQ ID NO: 38 (L2H1), SEQ ID NO: 31 and SEQ ID NO: 38 (L3H1), SEQ ID NO: 32 and SEQ ID NO: 38 (L4H1), SEQ ID NO: 33 and SEQ ID NO: 38 (L5H1), SEQ ID NO: 34 and SEQ ID NO: 38 (L6H1), SEQ ID NO: 35 and SEQ ID NO: 38 (L7H1), SEQ ID NO: 36 and SEQ ID NO: 38 (L8H1), or SEQ ID NO: 37 and SEQ ID NO: 38 (L9H1), or an expected phenotype thereof (e.g., IgA, IgG1, IgG2a, IgG2b, IgG3, IgM, IgE, or IgD), or an Fab or F(ab')2 fragment thereof.

In one embodiment, the antibody provided herein is an antibody comprising a combination of L1H1 (SEQ ID NO: 29 and SEQ ID NO: 38), L2H1 (SEQ ID NO: 30 and SEQ ID NO: 38), L6H1 (SEQ ID NO: 34 and SEQ ID NO: 38), L7H1 (SEQ ID NO: 35 and SEQ ID NO: 38), L8H1 (SEQ ID NO: 36 and SEQ ID NO: 38) or L9H1 (SEQ ID NO: 37 and SEQ ID NO: 38), or a class-switched antibody thereof (e.g., IgA, IgG1, IgG2a, IgG2b, IgG3, IgM, IgE, and IgD), or an Fab or F(ab')2 fragment thereof.

The antibody provided herein may comprise any of the constant regions known in the art. The light chain constant region may be, for example, a κ or λ light chain constant region, such as a mouse κ or λ light chain constant region. The heavy chain constant region may be, for example, an α, δ, ε, γ or µ heavy chain constant region, such as a mouse α, δ, ε, γ or µ heavy chain constant region. In one embodiment, the light or heavy chain constant region is a fragment, derivative, variant or mutant protein of a natural constant region.

In one embodiment, the antibody provided herein further comprises a human constant light chain kappa domain or a fragment thereof. The amino acid sequence of the light chain constant region is as follows: a human constant light chain kappa domain amino acid sequence: (SEQ ID NO: 49).

In one embodiment, the antibody provided herein further comprises a human heavy chain constant domain or a fragment thereof.

The heavy chain constant region amino acid sequence is as follows:
a human heavy chain constant region amino acid sequence (hIgG2): (SEQ ID NO: 50); and
a human heavy chain constant region amino acid sequence (hIgG4): (SEQ ID NO: 51).

In one embodiment, the GIPR antibody provided herein comprises a light chain domain amino acid sequence selected from V1 (SEQ ID NO: 103) listed herein. In one embodiment, the light chain domain amino acid sequence of the GIPR antibody differs from the light chain domain amino acid sequence of V1 by 15, 14, 13, 12, 11, 10, 9, 8, 7, 6, 5, 4, 3, 2, or 1 amino acid, wherein the difference in each sequence is independently a deletion, insertion or substitution of an amino acid residue. In another embodiment, the light chain domain amino acid sequence of the GIPR antibody is at least 70%, at least 75%, at least 80%, at least 85%, at least 90%, at least 95%, at least 97%, or at least 99% identical to the light chain domain amino acid sequence of V1. In another embodiment, the light chain domain polynucleotide coding sequence of the GIPR antibody is at least 70%, at least 75%, at least 80%, at least 85%, at least 90%, at least 95%, at least 97%, or at least 99% identical to the polynucleotide coding sequence of one of V1 and V2. In another embodiment, the light chain domain polynucleotide coding sequence of the GIPR antibody comprises a polynucleotide sequence that hybridizes to the complementary sequence of the polynucleotide coding sequence of the V1 light chain domain under moderately stringent conditions. In a further embodiment, the light chain domain polynucleotide coding sequence of the GIPR antibody comprises a polynucleotide sequence that hybridizes to the complementary sequence of the polynucleotide coding sequence of the V1 light chain domain under stringent conditions.

In one embodiment, the GIPR antibody provided herein comprises a heavy chain domain amino acid sequence selected from W1 (SEQ ID NO: 111), W2 (SEQ ID NO: 112) listed herein. In another embodiment, the heavy chain domain amino acid sequence of the GIPR antibody differs from the heavy chain domain sequence of one of W1 and W2 by 15, 14, 13, 12, 11, 10, 9, 8, 7, 6, 5, 4, 3, 2, or 1 amino acid, wherein the difference in each sequence is independently a deletion, insertion or substitution of an amino acid residue. In another embodiment, the heavy chain domain amino acid sequence of the GIPR antibody is at least 70%, at least 75%, at least 80%, at least 85%, at least 90%, at least 95%, at least 97%, or at least 99% identical to the heavy chain domain sequence of one of W1 and W2. In another embodiment, the heavy chain domain polynucleotide coding sequence of the GIPR antibody is at least 70%, at least 75%, at least 80%, at least 85%, at least 90%, at least 95%, at least 97%, or at least 99% identical to the polynucleotide coding sequence of one of W1 and W2. In another embodiment, the heavy chain domain polynucleotide coding sequence of the GIPR antibody comprises a polynucleotide sequence that hybridizes to the complementary sequence of the polynucleotide coding sequence of one of W1 and W2 heavy chain domains under moderately stringent conditions. In one embodiment, the heavy chain domain polynucleotide coding sequence of the GIPR antibody comprises a polynucleotide sequence that hybridizes to the complementary sequence of the polynucleotide coding sequence of one of W1 and W2 heavy chain domains under stringent conditions.

In another embodiment, the antibody provided herein is an antibody comprising a combination V1W1 (SEQ ID NO: 103 and SEQ ID NO: 111), or V1W2 (SEQ ID NO: 103 and SEQ ID NO: 112).

In one embodiment, the GIPR antibody provided herein is selected from a murine-derived antibody, a humanized antibody, a chimeric antibody, a monoclonal antibody, a polyclonal antibody, a recombinant antibody, an antigen binding antibody fragment, a single-chain antibody, a diabody, a triabody, a tetrabody, an Fab fragment, an F(ab')x fragment, a domain antibody, an IgD antibody, an IgE antibody, an IgM antibody, an IgG1 antibody, an IgG2 antibody, an IgG3 antibody or an IgG4 antibody.

In one embodiment, the GIPR antibody provided herein is a GIPR monoclonal antibody.

In another embodiment, the GIPR antibody provided herein is a monoclonal antibody comprising a combination of amino acid sequences selected from the list below: SEQ ID NO: 29 and SEQ ID NO: 38, SEQ ID NO: 30 and SEQ ID NO: 38, SEQ ID NO: 31 and SEQ ID NO: 38, SEQ ID NO: 32 and SEQ ID NO: 38, SEQ ID NO: 33 and SEQ ID NO: 38, SEQ ID NO: 34 and SEQ ID NO: 38, SEQ ID NO: 35 and SEQ ID NO: 38, SEQ ID NO: 36 and SEQ ID NO: 38, and SEQ ID NO: 37 and SEQ ID NO: 38.

In one embodiment, the GIPR antibody provided herein is a murine-derived GIPR antibody. In another embodiment, the GIPR antibody provided herein is a humanized GIPR antibody.

In one embodiment, the GIPR antibody provided herein reduces human GIP signaling with an IC₅₀ value of about 1 nM to about 200 nM or about 1 nM to about 100 nM.

### Antibody and antibody fragment

In one embodiment, the antibody provided herein is an intact antibody (including polyclonal, monoclonal, chimeric, humanized or human antibodies having full length heavy and/or light chains). In another embodiment, the antibody provided herein is an antibody fragment, for example, an F(ab')₂, Fab, Fab', Fv, Fc, or Fd fragment, a single domain antibody, a single-chain antibody, a maxibody, a minibody, an intrabody, a diabody, a triabody, a tetrabody, v-NAR or bis-scFv (see, for example, Hollinger and Hudson, 2005, Nature Biotechnology, 23:1126-1136). In another embodiment, the antibody provided herein includes the antibody polypeptide as disclosed in U.S. Pat. No. 6703199, including a fibronectin polypeptide monoclonal antibody. In a further embodiment, the antibody provided herein includes the single-chain polypeptide as disclosed in U.S. Patent Publication 2005/0238646.

In one embodiment, the variable region of a gene of a monoclonal antibody expressed in hybridomas is amplified using nucleotide primers. These primers can be synthesized by one of ordinary skill in the art or purchased from commercial sources. The primers for murine and human variable regions, including primers for V_{Ha}, V_{Hb}, V_{Hc}, V_{Hd}, C_{H1}, V_{L}, and C_{L} regions, can be purchased from commercial sources. These primers can be used to amplify heavy or light chain variable regions, which can then be inserted into vectors for example IMMUNOZAP^{™}H or IMMUNOZAP^{™}L (Stratagene). These vectors can then be introduced into E. coli, yeast, or mammalian-based expression systems. These methods can be used to produce large amounts of single-chain proteins comprising a fusion of V_{H} and V_{L} domains (see Bird et al., 1988, Science 242:423-426).

It should be understood by one skilled in the art that certain proteins, such as antibodies, can undergo a variety of post-transcriptional modifications. The types and extents of these modifications depend on the host cell lines used to express the protein as well as the culture conditions. Such modifications include variations in glycosylation, methionine oxidation, diketopiperazine formation, aspartate isomerization and asparagine deamidation. Carboxy-terminal basic residues (for example lysine or arginine) of the antibody may be lost due to frequent modification actions of carboxypeptidases (see Harris, 1995, Journal of Chromatography 705:129-134).

Murine monoclonal antibodies can be produced using common hybridoma cell methods. The monoclonal antibody can be isolated and purified by a variety of established techniques. Such isolation techniques include affinity chromatography with Protein A-sepharose, size exclusion chromatography, and ion exchange chromatography, (see, e.g., Coligan, pages 2.7.1-2.7.12 and pages 2.9.1-2.9.3; Baines et al., "Purification of Immunoglobulin G (IgG)," Methods in Molecular Biology, vol. 10, pages 79-104 (The Humana Press, Inc., 1992)). The monoclonal antibody can be purified by affinity chromatography using appropriate ligands screened on the basis of particular properties of the antibody (e.g., heavy or light chain isotype, binding specificity, etc.). Examples of appropriate ligands of affinity chromatography include Protein A, Protein G, an anti-constant region (light chain or heavy chain) antibody, an anti-idiotypic antibody, and an FGF21 binding protein, or a fragment or variant thereof.

Antibodies with increased affinity, for example antibodies with increased affinity for c-erbB-2, can be obtained by modifying the molecule for affinity maturation using complementary determining regions (CDRs) in the center of the antibody binding site (Schier et al., 1996. J. Mol. Biol. 263:551-567). Accordingly, such techniques are useful in preparing an antibody against a human GIPR.

Antibodies against a human GIPR can be used, for example, in assays to detect the presence or absence of the human GIPR, either in vitro or in vivo.

Antibodies can also be prepared by any of the traditional techniques. For example, these antibodies can be purified from cells that naturally express them (e.g., an antibody can be purified from a hybridoma that produces it) or produced in recombinant expression systems using any technique known in the art. See, for example, Monoclonal Antibodies, Hybridomas: A New Dimension in Biological Analyses, Kennet et al. (eds.), Plenum Press, (1980); and Antibodies: A Laboratory Manual, Harlow and Land (eds.), Cold Spring Harbor Laboratory Press (1988). This is discussed in the nucleic acid section below.

Antibodies can be prepared and screened for desired properties by any known techniques. Some techniques relate to the isolation of nucleic acids encoding polypeptide chains (or portions thereof) of related antibodies (e.g., anti-GIPR antibodies) and the manipulation of the nucleic acids by recombinant DNA techniques. The nucleic acids can be fused with another relevant nucleic acid or modified (e.g., by mutagenesis or other traditional techniques) to add, delete or substitute one or more amino acid residues.

When it is desired to improve the affinity of antibodies comprising one or more of the above-mentioned CDRs herein, a variety of affinity maturation protocols can be used, including maintenance of CDRs (Yang et al., 1995, J. Mol. Biol. 254: 392-403), chain shuffling (Marks et al., 1992, Bio/Technology 10:779-783), use of mutant strains of E. coli (Low et al., 1996, J. Mol. Biol. 250:350-368), DNA rearrangement (Patten et al., 1997, Curr. Opin. Biotechnol. 8:724-733), phage display (Thompson et al., 1996, J. Mol. Biol. 256:7-88) and additional PCR techniques (Crameri et al., 1998, Nature 391: 288-291). All of these affinity maturation methods are discussed in Vaughan et al., 1998, Nature Biotechnology, 16:535-539.

In one embodiment, the antibody provided herein is a anti-GIPR fragment. The fragment may consist entirely of antibody-derived sequences or may comprise additional sequences. Examples of antigen binding fragments include Fab, F(ab')2, single-chain antibodies, diabodies, triabodies, tetrabodies and domain antibodies, and other examples are provided in Lunde et al., 2002, Biochem. Soc. Trans. 30:500-06.

Heavy and light chain variable domains (Fv regions) can be linked by an amino acid bridge (short peptide linker) to form a single-chain antibody, resulting in a single polypeptide chain. Such single-chain Fvs (scFvs) have been prepared by fusing DNA encoding a peptide linker between DNAs encoding the two variable domain polypeptides (V_{L} and V_{H}). The resulting polypeptides can fold back on themselves to form antigen binding monomers, or they can form polymers (e.g., dimers, trimers or tetramers), depending on the length of a flexible linker between the two variable domains (Kortt et al., 1997, Prot. Eng. 10:423; Kortt et al., 2001, Biomol. Eng. 18:95-108). By combining different V_{L} and V_{H}-comprising polypeptides, polymeric scFvs that bind to different phenotypes can be formed (Kriangkum et al., 2001, Biomol. Eng. 18:31-40). Techniques developed for the production of single chain antibodies include those described in U.S. Pat. No. 4946778; Bird, 1988, Science 242:423; Huston et al., 1988, Proc. Natl. Acad. Sci. USA 85:5879-5883; Ward et al., 1989, Nature 334:544-546; de Graaf et al., 2002, Methods Mol Biol. 178:379-87. Single-chain antibodies derived from antibodies provided herein including, but not limited to, scFvs comprising the variable domain combination L1H1, are encompassed within the scopes described herein.

Antigen binding fragments derived from antibodies can also be obtained by proteolysis of the antibodies, for example, by pepsin or papain digestion of intact antibodies according to traditional methods. By way of example, antibody fragments can be produced by enzymatic cleavage of antibodies with pepsin to provide a SS fragment referred to as F(ab')2. This fragment can be further cleaved using a sulfhydryl reducing agent to produce 3.5S Fab' monovalent fragments. An alternative scheme is to perform the cleavage reaction with a sulfhydryl protecting group, resulting in the cleavage of the disulfide bond; in addition, enzymatic cleavage with papain directly produces two monovalent Fab fragments and an Fc fragment. These methods are described, for example, in Goldenberg, U.S. Pat. No. 4,331,647, Nisonoff et al., 1960, Arch. Biochem. Biophys. 89:230; Porter, 1959, Biochem. J. 73:119; Edelman et al., Methods in Enzymology I:422, (Academic Press, 1967); and Andrews and Titus, J.A. Current Protocols in Immunology (Coligan et al., (eds), John Wiley & Sons, 2003), pages 2.8,1-2.8.10 and pages 2.10A.1-2.10A.5. Other methods for cleaving antibodies, such as preparing heavy chains to form monovalent heavy and light chain fragments (Fds), further cleaving of fragments, or other enzymatic, chemical or genetic techniques can also be used, provided that the fragments bind to the antigen that is recognized by the intact antibody.

Another form of an antibody fragment is a peptide comprising one or more complementarity determining regions (CDRs) of an antibody. CDRs can be obtained by constructing polypeptides that encode the relevant CDRs. Such polypeptides can be prepared, for example, by using the polymerase chain reaction to synthesize the variable region using mRNA of antibody-producing cells as a template, see, for example, Larrick et al., 1991, Methods: A Companion to Methods in Enzymology 2:106; Courtenay-Luck, "Genetic Manipulation of Monoclonal Antibodies," Monoclonal Antibodies: Production, Engineering and Clinical Application, Ritter et al., (eds.), page 166 (Cambridge University Press, 1995); and Ward et al., "Genetic Manipulation and Expression of Antibodies," Monoclonal Antibodies: Principles and Applications, Birch et al., (eds.), page 137 (Wiley-Liss, Inc., 1995). The antibody fragment can further comprise at least one variable domain of the antibody described herein. Thus, for example, the V region domain can be monomeric and be a V_{H} or V_{L} domain, which can independently bind to GIPR with an affinity of at least 1 × 10⁻⁷ M or higher as described below.

The variable region domain can be any natural variable domain or a genetically engineered version thereof. Genetically engineered version refers to a variable region domain produced by recombinant DNA engineering techniques. Such genetically engineered versions include those generated, for example, from a specific antibody variable region by insertions, deletions, or alterations in the amino acid sequences of the specific antibody. Particular examples include variable region domains comprising only one CDR and optionally one or more framework amino acids from one antibody and the remainder of the variable region domain from another antibody which are assembled by genetic engineering.

The variable region domain can be covalently linked at the C-terminal amino acid to at least one other antibody domain or a fragment thereof. Thus, by way of example, a V_{H} domain that is present in the variable region domain can be linked to an immunoglobulin C_{H1} domain or a fragment thereof. Similarly, a V_{L} domain can be linked to a C_{K} domain or a fragment thereof. In this way, for example, the antibody can be an Fab fragment, wherein the antigen-binding domain contains combined V_{H} and V_{L} domains covalently linked at the C-terminal thereof to C_{H1} and C_{K} domains, respectively. The C_{H1} domain can be extended with further amino acids, for example to provide a hinge region or a portion of a hinge region domain as found in an Fab' fragment, or to provide further domains, such as antibody C_{H2} and C_{H3} domains.

### Derivatives and variants of antibodies

The nucleotide sequences encoding the amino acid sequence L1 and H can be altered, for example, by random mutagenesis or by site-directed mutagenesis (e.g., oligonucleotide-induced site-directed mutagenesis) to generate an altered polynucleotide comprising one or more specific nucleotide substitutions, deletions, or insertions as compared to the non-mutated polynucleotide. Examples of techniques for making such alterations are described in Walder et al., 1986, Gene 42:133; Bauer et al., 1985, Gene 37:73; Craik, 1985, BioTechniques, 3:12-19; Smith et al., 1981, Genetic Engineering: Principles and Methods, Plenum Press; and U.S. Pat. Nos. 4518584 and 4737462. These and other methods can be used to produce, for example, derivatives of anti-GIPR antibodies with desired properties, such as enhanced affinity, avidity or specificity for a GIPR, enhanced in vivo or in vitro activity or stability, or reduced in vivo side effects as compared to the underivatized antibody.

Other derivatives of anti-GIPR antibodies in the art include covalent or aggregated conjugates of anti-GIPR antibodies or fragments thereof, with other proteins or polypeptides, for example, by expression of recombinant fusion proteins comprising heterologous polypeptides fused to the N-terminus or C-terminus of an anti-GIPR antibody polypeptide. For example, the binding peptide can be a heterologous signal (or leader) polypeptide, e.g., a yeast α factor leader peptide or an epitope-tagged peptide. An antibody comprising fusion proteins can comprise peptides added to facilitate purification or identification of the antibody (e.g., polyhistidine). An antibody can also be linked to a FLAG peptide as described in Hopp et al., 1988, Bio/Technology 6:1204 and U.S. Pat. No. 5011912. The FLAG peptide is highly antigenic and provides an epitope reversibly bound by a specific monoclonal antibody (mAb), enabling rapid detection and facile purification of an expressed recombinant protein. Reagents useful for preparing fusion proteins in which the FLAG peptide is fused to a given polypeptide are commercially available (Sigma-Aldrich, St. Louis, MO). In another embodiment, oligomers that comprise one or more antibodies can be employed as GIPR antagonists or higher-order oligomers. Oligomers can be in the form of covalently linked or non-covalently linked dimers, trimers, or higher-order oligomers. Oligomers comprising two or more antibodies may be used, with one example being a homodimer. Other oligomers include heterodimers, homotrimers, heterotrimers, homotetramers, heterotetramers, etc.

One embodiment is directed to oligomers comprising multiple antibodies, which are linked via covalent or non-covalent interactions between the peptide moieties fused to the antibodies. Such peptides may be peptide linkers (spacers), or peptides that have the property of promoting oligomerization. Leucine zippers and certain polypeptides derived from antibodies are peptides that can promote oligomerization of antibodies, as described in detail below.

In specific embodiments, oligomers comprise from two to four antibodies. The antibodies of the oligomers can be in any form, such as any of the forms described above, e.g., variants or fragments. Preferably, the oligomers comprise antibodies having GIPR binding activity.

In one embodiment, oligomers are prepared using polypeptides derived from immunoglobulins. Preparation of fusion proteins comprising certain heterologous polypeptides fused to various portions of antibody-derived polypeptides (including the Fc domain) has been described, e.g., in Ashkenazi et al., 1991, PNAS USA 88:10535; Byrn et al., 1990, Nature 344:677; and Hollenbaugh et al., Construction of Immunoglobulin Fusion Proteins, Current Protocols in Immunology, Suppl. 4, pages 10.19.1-10.19.11. One embodiment herein is directed to a dimer comprising two fusion proteins produced by fusing an GIP binding fragment of an anti-GIPR antibody to the Fc region of the antibody. The dimer can be prepared in the following ways: for example, inserting a fusion gene encoding a fusion protein into an appropriate expression vector, then expressing the fusion gene in host cells transformed with the recombinant expression vector, and allowing the expressed fusion protein to assemble like an antibody molecule, whereupon the inter-chain disulfide bonds between the Fc moieties form a dimer.

The term "Fc polypeptide" as used herein includes polypeptides in the form of natural proteins and mutant proteins derived from the Fc region of antibodies. Truncated forms of such polypeptides comprising the hinge region that promotes dimerization are also included. Fusion proteins comprising Fc moieties (and oligomers formed therefrom) offer the advantage of facile purification by affinity chromatography over Protein A or Protein G columns.

An appropriate Fc polypeptide, described in PCT application WO 93/10151 (incorporated herein by reference), is a single-chain polypeptide extending from the N-terminal hinge region to the natural C-terminus of the Fc region of a human IgG1 antibody. Another useful Fc polypeptide is the Fc mutant protein as described in U.S. Pat. No. 5457035 and in Baum et al., 1994, EMBO J. 13:3992-4001. The amino acid sequence of this mutant protein is identical to that of the natural Fc sequence as shown in WO 93/10151, except that amino acid 19 has been changed from leucine to alanine, amino acid 20 has been changed from leucine to glutamine, and amino acid 22 has been changed from glycine to alanine. The mutant protein exhibits reduced affinity for Fc receptors. In other embodiments, the heavy chain and/or light chain of an anti-GIPR antibody may be substituted with the variable portion of the heavy chain and/or light chain thereof.

Alternatively, the oligomer is a fusion protein comprising multiple antibodies, with or without peptide linkers (spacer peptides). These appropriate peptide linkers are described in U.S. Pat. No. 4751180 and 4935233.

Another method for preparing oligomeric antibodies involves use of a leucine zipper. Leucine zipper domains are peptides that promote oligomerization of the proteins in which they are present. Leucine zippers were originally identified in several DNA-binding proteins (Landschulz et al., 1988, Science 240:1759), and later found in a variety of different proteins. Among the known leucine zippers are natural peptides or derivatives thereof that can be dimerized or trimerized. Examples of leucine zipper domains suitable for producing soluble oligomeric proteins are described in PCT application WO 94/10308, and the leucine zippers derived from lung surfactant protein D (SPD) are described in Hoppe et al., 1994, FEBS Letters 344:191, incorporated herein by reference. The use of a modified leucine zipper that allows for stable trimerization of a heterologous protein fused thereto is described in Fanslow et al., 1994, Semin. Immunol. 6:267-78. In one method, recombinant fusion proteins comprising an anti-GIPR antibody fragment or a derivative thereof fused to a leucine zipper peptide are expressed in appropriate host cells, and a soluble oligomeric anti-GIPR antibody fragment or a derivative thereof is collected from the culture supernatant.

In another embodiment, the antibody derivative can comprise at least one of the CDRs disclosed herein. By way of example, one or more CDRs can be integrated into known antibody framework regions (IgG1, IgG2, etc.), or bind to appropriate vectors to enhance the half-life thereof. Appropriate vectors include, but are not limited to Fc, albumin, transferrin, etc. These and other appropriate vectors are known in the art. Such CDR conjugated peptides can be in monomeric, dimeric, tetrameric, or other forms. In one embodiment, one or more water-soluble polymers bind at one or more specific sites, for example at the amino terminus, of a binding agent. In an example, an antibody derivative comprises one or more water-soluble polymer attachments, including, but not limited to, polyethylene glycol, polyoxyethylene glycol, or polypropylene glycol. See, for example, U.S. Pat. Nos. 4640835, 4496689, 4301144, 4670417, 4791192 and 4179337. In some embodiments, the derivative comprises one or more of monomethoxy-polyethylene glycol, dextran, cellulose or other carbohydrate-based polymers, poly(N-vinyl pyrrolidone)-polyethylene glycol, polyoxyethylated polyols (e.g., glycerol) and polyvinyl alcohol, and mixtures of such polymers. In some embodiments, one or more water-soluble polymers are randomly attached to one or more side chains. In some embodiments, PEG can act to improve the therapeutic effect of a binding agent, such as an antibody. Certain such methods are described, for example, in U.S. Pat. No. 6133426, which is incorporated herein by reference for any purpose.

It should be understood that the antibody provided herein may have at least one amino acid substitution, provided that the antibody retains the binding specificity. Therefore, modifications of antibody structures are encompassed within the scopes described herein. These modifications can include amino acid substitutions, which may be conservative or non-conservative, that do not destroy the GIPR binding ability of an antibody. Conservative amino acid substitutions may include non-natural amino acid residues, which are generally integrated by chemical peptide synthesis rather than by synthesis in biological systems. These amino acid residues include peptidomimetics and other reversed or inverted forms of amino acid moieties. Conservative amino acid substitutions can also involve a substitution of a natural amino acid residue with a non-natural residue such that there is little or no effect on the polarity or charge of the amino acid residue at that site. Non-conservative substitutions can involve the exchange of a member of one class of amino acids or amino acid analogs for a member from another class with different physical properties (e.g., size, polarity, hydrophobicity and charge).

Moreover, one skilled in the art may generate variants to be tested, which contain an amino acid substitution at each desired amino acid residue. The variants can be screened using activity assays known to one skilled in the art. Such variants can be used to gather information about appropriate variants. By way of example, if it is found that a change in a certain amino acid residue results in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

One skilled in the art will be able to determine appropriate variants of the polypeptide as listed herein using known techniques. In some embodiments, one skilled in the art may identify appropriate regions of the molecule that may be altered without destroying the activity by targeting regions not to be important for activity. In some embodiments, residues or portions of molecules that are conserved among similar polypeptides can be identified. In some embodiments, even conservative substitutions for regions that are important for biological activity or structure do not destroy the biological activity or have an adverse effect on the polypeptide structure. Additionally, one skilled in the art can review structure- function studies to identify residues in similar polypeptides that are important for activity or structure. In view of such a comparison, the importance of amino acid residues in a protein that correspond to amino acid residues which are important for activity or structure in similar proteins can be predicted. One skilled in the art may choose chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of an antibody with respect to the three-dimensional structure thereof. In some embodiments, one skilled in the art may choose not to make significant alteration to amino acid residues predicted to be on the surface of a protein, because such residues may be involved in important interactions with other molecules. A number of scientific publications have been devoted to the prediction of secondary structure. See, Moult, 1996, Curr. Op. Biotech. 7:422-427, Chou et al., 1974, Biochemistry 13:222-245; Chou et al., 1974, Biochemistry 113:211-222; Chou et al., 1978, Adv. Enzymol. Relat. Areas Mol. Biol. 47:45-148; Chou et al., 1979, Ann. Rev. Biochem. 47:251-276, and Chou et al., Biophys. J. 26:367-384. Moreover, computer programs are currently available to assist with predicting secondary structure. By way of example, two polypeptides or proteins which have a sequence identity greater than 30%, or similarity greater than 40% often have similar higher-order structures. The recent growth of the Protein Data Bank (PDB) has provided enhanced predictability of secondary structure, including the potential number of folds within the structure of a polypeptide or protein. See Holm et al., 1999, Nucl. Acid. Res. 27:244-247. It has been suggested (Brenner et al., 1997, Curr. Op. Struct. Biol. 7:369-376) that there are a limited number of folds in a given polypeptide or protein and that once a critical number of structures are determined, the structural prediction will become dramatically more accurate.

Additional methods for predicting secondary structure include "threading" (Jones, 1997, Curr. Opin. Struct. Biol., 7:377-87; Sippl et al., 1996, Structure 4:15-19); "profile analysis" (Bowie et al., 1991, Science 253:164-170; Gribskov et al., 1990, Meth. Enzym. 183:146-159; Gribskov et al., 1987, Proc. Nat. Acad. Sci. USA 84:4355-4358), and "evolutionary linkage" (see Holm, supra (1999), and Brenner, supra (1997)). In some embodiments, antibody variants include glycosylation variants, wherein the number and/or type of glycosylation sites have been altered compared to the amino acid sequences of a parent polypeptide. In some embodiments, variants comprise a greater or lesser number of N-linked glycosylation sites than natural proteins. Alternatively, a substitution that removes this sequence may remove an existing N-linked carbohydrate chain. Also provided is a rearrangement of N-linked carbohydrate chains, wherein one or more N-linked carbohydrate chain sites (generally those that are naturally occurring) are removed and one or more new N-linked sites are created. Additional preferred antibody variants include cysteine variants, wherein one or more cysteine residues are deleted or substituted with another amino acid (e.g., serine) as compared to the parent amino acid sequence. Cysteine variants can be useful when antibodies must be folded into a biologically active conformation (for example, after isolation of soluble inclusion bodies). Cysteine variants generally have fewer cysteine residues than natural proteins, and generally have an even number of cysteines to minimize interactions resulting from unpaired cysteines.

Desired amino acid substitutions (whether conservative or non-conservative) can be determined by one skilled in the art at the time such substitutions are desired. In some embodiments, amino acid substitutions can be used to identify important residues of human GIPR antibodies, or to increase or decrease the affinity of the human GIPR antibodies described herein.

According to some embodiments, preferred amino acid substitutions are those which: (1) reduce susceptibility to proteolysis, (2) reduce susceptibility to oxidation, (3) alter the binding affinity for forming protein complexes, (4) alter the binding affinity, and/or (4) confer or modify other physiochemical or functional properties on such polypeptides. According to some embodiments, single or multiple amino acid substitutions (in some embodiments, conservative amino acid substitutions) may be performed in naturally occurring sequences (in some embodiments, in the portion of the polypeptide outside the domains that form intermolecular contacts). In some embodiments, conservative amino acid substitutions generally cannot substantially alter the structural characteristics of a parent sequence (e.g., the substitution of amino acids shall not destroy a helix present in the parent sequence or interfere with other types of secondary structure that characterize the parent sequence). Examples of art-recognized polypeptide secondary and tertiary structures are described in Proteins, Structures and Molecular Principles, Creighton (eds.), W.H. Freeman and Company (1984); Introduction to Protein Structure, Branden and Tooze (eds.), Garland Publishing (1991); and Thornton et al., 1991, Nature 354:105, each of which is incorporated herein by reference.

In some embodiments, the antibody provided herein can be chemically bonded with polymers, lipids, or other moieties.

An antigen binding agent may comprise at least one of the CDRs described herein incorporated into a biocompatible framework structure. In an example, the biocompatible framework structure comprises a polypeptide or a portion thereof that is sufficient to form a conformationally stable structural support, or framework, or scaffold, which is able to present one or more amino acid sequences (e.g., CDRs, variable regions, etc.) that bind to an antigen in a localized surface region. Such structures can be naturally occurring polypeptides or polypeptide "folds" (structural motifs), or can have one or more modifications, such as additions, deletions or substitutions of amino acids, relative to a naturally occurring polypeptide or fold. These scaffolds can be derived from a polypeptide of any species (or of more than one species), such as humans, other mammals, other vertebrates, invertebrates, bacteria or viruses.

Generally, biocompatible framework structures are based on protein scaffolds or frameworks rather than immunoglobulin domains. By way of example, those protein scaffolds based on fibronectin, ankyrin, lipocalin, neocarzinostatin, cytochrome b, CP1 zinc finger protein, PST1, coiled-coil protein, LACI-D1, Z domain and tendamistat domain can be used (see, for example, Nygren and Uhlen, 1997, Current Opinion in Structural Biology 7:463-469).

Additionally, one skilled in the art will recognize that appropriate binding agents include portions of these antibodies, such as one or more of the heavy chain CDR1s, CDR2s and CDR3s, and light chain CDR1s, CDR2s and CDR3s as specifically disclosed herein. At least one of the heavy chain CDR1, CDR2, CDR3, light chain CDR1, CDR2 and CDR3 regions has at least one amino acid substitution, provided that the antibody retains the binding specificity of the non-substituted CDR. The non-CDR portion of the antibody may be a non-protein molecule, wherein the binding agent cross-blocks the binding of the antibody disclosed herein to human GIPR and/or inhibits GIP signaling via the receptor. The non-CDR portion of the antibody may be a non-protein molecule, wherein the antibody exhibits a binding type similar to that of at least one of the antibodies L1H1/L8H1 to human GIP peptide, and/or neutralizes the activity of GIP in a competitive binding assay. The non-CDR portion of the antibody may be composed of amino acids, wherein the antibody is a recombinant binding protein or a synthetic peptide, and the recombinant binding protein cross-blocks the binding of the antibody disclosed herein to human GIPR and/or neutralizes GIP activity in vivo or in vitro. The non-CDR portion of the antibody may be composed of amino acids, wherein the antibody is a recombinant antibody, and the recombinant antibody exhibits a binding type similar to that of at least one of the antibodies L1H1/L8H1 to human GIPR peptide, and/or neutralizes GIP signaling in a competitive binding assay.

### A fusion protein of a GIPR antibody and FGF21

In one embodiment, provided herein is a fusion protein of a GIPR antibody and FGF21, wherein the fusion protein comprises an antibody capable of specifically binding to a GIPR, and one, two, three, four, five, six, seven, or eight FGF21 fragments; in the fusion protein, the amino terminus of an FGF21 fragment is linked to the carboxy terminus of a GIPR antibody light or heavy chain via a peptide linker sequence (Linker).

In one embodiment, provided herein is a fusion protein of a GIPR antibody and FGF21, wherein the fusion protein comprises an antibody capable of specifically binding to a GIPR, and one, two, three, or four FGF21 fragments; in the fusion protein, the amino terminus of an FGF21 fragment is linked to the carboxy terminus of a GIPR antibody light or heavy chain via a peptide linker sequence (Linker).

In one embodiment, provided herein is a fusion protein of a GIPR antibody and FGF21, wherein the fusion protein comprises an antibody capable of specifically binding to a GIPR, and one, or two FGF21 fragments; in the fusion protein, the amino terminus of an FGF21 fragment is linked to the carboxy terminus of a GIPR antibody light or heavy chain via a peptide linker sequence (Linker).

In another embodiment, provided herein is an FGF21 fusion protein, wherein the fusion protein comprises a GIPR antibody and one or two FGF21 fragments; in the fusion protein, the amino terminus of an FGF21 fragment is linked to the carboxy terminus of a GIPR antibody light chain via a peptide linker sequence (Linker): N'-R-Linker-FGF21-C'; or the amino terminus of an FGF21 fragment is linked to the carboxy terminus of a GIPR antibody heavy chain: N'-R-Linker-FGF21-C'; wherein N' represents the amino terminus of a polypeptide chain of the fusion protein, C' represents the carboxy terminus of a polypeptide chain of the fusion protein, FGF21 represents an FGF21 fragment, R is the amino acid sequence of the light or heavy chain of the GIPR antibody, and Linker represents a peptide linker sequence.

In one embodiment, in the FGF21 fusion protein provided herein, the FGF21 fragments are each independently selected from one of the following amino acid sequences: SEQ ID NO: 77, SEQ ID NO: 78, SEQ ID NO: 79, SEQ ID NO: 80, and SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 115, SEQ ID NO: 116, SEQ ID NO: 117, SEQ ID NO: 118 and SEQ ID NO: 122.

In one embodiment, in the FGF21 fusion protein provided herein, each of the sequences of the peptide linker (Linker) independently comprises from 1 to 200 amino acid amines, from 2 to 100 amino acid amines, from 5 to 50 amino acid amines, from 6 to 25 amino acid amines, or from 10 to 20 amino acid amines.

In another embodiment, in the FGF21 fusion protein provided herein, the sequences of the peptide linker (Linker) are each independently selected from the following amino acid sequences: SEQ ID NO: 61, SEQ ID NO: 62, and SEQ ID NO: 63, SEQ ID NO: 64, SEQ ID NO: 65, SEQ ID NO: 66, SEQ ID NO: 67 and SEQ ID NO: 68.

### Nucleic acids

In one aspect, provided herein is an isolated nucleic acid molecule. The nucleic acid molecule comprises, for example, polynucleotides that encode all or part of an antibody, such as one or both chains of the antibody or FGF21 fusion protein herein, or a fragment, derivative, mutant protein or variant thereof; polynucleotides sufficient for use as hybridization probes; PCR primers or sequencing primers for identifying, analyzing, mutating or amplifying a polynucleotide encoding a polypeptide; anti-sense nucleic acids for inhibiting expression of a polynucleotide, and complementary sequences thereof. The nucleic acid may be in any length. The nucleic acid may be, for example, 5, 10, 15, 20, 25, 30, 35, 40, 45, 50, 75, 100, 125, 150, 175, 200, 250, 300, 350, 400, 450, 500, 750, 1000, 1500, 3000, 5000 or more nucleotides in length, and/or comprise one or more additional sequences, for example, regulatory sequences, and/or be one portion of a larger nucleic acid, for example, a vector. The nucleic acid may be single-stranded or double-stranded and comprise RNA and/or DNA nucleotides, and artificial variants thereof (e.g., peptide nucleic acids).

A nucleic acid encoding an antibody polypeptide (e.g., heavy or light chain, variable domain only, or full length) can be isolated from B-cells of mice that have been immunized with a GIPR antigen. The nucleic acid of the antibody or FGF21 fusion protein can be isolated by conventional methods such as polymerase chain reaction (PCR).

The nucleic acid sequences encoding the heavy and light chain variable regions are shown above. The skilled artisan will appreciate that, due to the degeneracy of the genetic code, each of the polypeptide sequences disclosed herein can be encoded by a large number of other nucleic acid sequences. Provided herein is each degenerate nucleotide sequence encoding the antibody or FGF21 fusion protein provided herein.

Further provided herein are nucleic acids that hybridize to other nucleic acids (e.g., nucleic acids comprising a nucleotide sequence of any of L1H1/L8H1) under specific hybridization conditions. Methods for hybridizing nucleic acids are well-known in the art. See, for example, Current Protocols in Molecular Biology, John Wiley & Son (1989), 6.3.1-6.3.6. As defined herein, for example, moderately stringent conditions use a prewashing solution containing 5x sodium chloride/sodium citrate (SSC), 0.5% SDS, 1.0 mM EDTA (pH 8.0), a hybridization buffer of approximately 50% formamide, 6x SSC, a hybridization temperature of 55°C (or other similar hybridization solutions, such as a solution containing approximately 50% formamide, with a hybridization temperature of 42°C), and elution conditions of 60°C, 0.5x SSC and 0.1% SDS. Stringent hybridization conditions use hybridization in 6x SSC at 45°C, followed by one or more washes in 0.1x SSC and 0.2% SDS at 68°C. Furthermore, one skilled in the art can manipulate hybridization and/or washing conditions to increase or decrease the stringency of hybridization such that nucleic acids comprising nucleotide sequences that are at least 65%, 70%, 75%, 80%, 85%, 90%, 95%, 98% or 99% homologous to each other generally remain hybridized to each other. The basic parameters affecting the choice of hybridization conditions and guidance for designing appropriate conditions are listed in, for example, Sambrook, Fritsch and Maniatis, 1989, Molecular Cloning: A Laboratory Manual, Cold Spring Harbor Laboratory Press, chapters 9 and 11, and Current Protocols in Molecular Biology, 1995, Ausubel et al., eds., John Wiley & Sons, Inc., sections 2.10 and 6.3-6.4, and can be readily determined by one of ordinary skill in the art based on, for example, the length and/or base composition of the DNA. Changes can be introduced by mutations into a nucleic acid, thereby leading to changes in the amino acid sequence of the polypeptide (e.g., an antigen binding protein) encoded thereby. Mutations can be introduced using any technique known in the art. In one embodiment, one or more specific amino acid residues are altered using, for example, a site-directed mutagenesis protocol. In another embodiment, one or more randomly selected residues are altered using, for example, a random mutagenesis protocol. No matter how it is made, a mutant polypeptide can be expressed and screened for desired properties.

Mutations can be introduced into a nucleic acid without significantly altering the biological activity of a polypeptide encoded thereby. For example, nucleotide substitutions leading to amino acid substitutions at non-essential amino acid residues can be made. In one embodiment, nucleotide sequences provided herein for L1 to L9 and H1 or the FGF21 fusion protein, or fragments, variants, or derivatives thereof, are mutated such that the amino acid sequence encoded thereby comprises, as compared to that encoded by L1 to L9 and H as shown herein, one or more deletions or substitutions of amino acid residues, resulting in sequences involving two or more different residues. In another embodiment, mutagenesis leads to insertion of an amino acid adjacent to one or more amino acid residues of L1 to L9 and H1 or the FGF21 fusion protein as shown herein, resulting in sequences involving two or more different residues. Alternatively, one or more mutations can be introduced into a nucleic acid to selectively alter the biological activity (e.g., binding to GIPR) of a polypeptide encoded thereby. For example, the mutation can quantitatively or qualitatively change the biological activity. Examples of quantitative changes include increasing, reducing or eliminating the activity. Examples of qualitative changes include altering the antigen specificity of the antibody or FGF21 fusion protein.

In another aspect, provided herein are nucleic acid molecules that are suitable for use as primers or hybridization probes for the detection of nucleic acid sequences herein. The nucleic acid molecule herein can comprise only a portion of a nucleic acid sequence encoding the full-length polypeptide herein, for example, a fragment that can be used as a probe or a primer or a fragment encoding an active portion (e.g., a GIPR binding portion) of the polypeptide herein.

Probes based on the nucleic acid sequence herein can be used to detect the nucleic acid or similar nucleic acids, for example, transcripts encoding the polypeptide herein. The probes may comprise a labelling group, e.g., a radioisotope, a fluorescent compound, an enzyme, or an enzyme cofactor. Such probes can be used to identify a cell that expresses the polypeptide.

In another aspect, provided herein is a vector comprising a nucleic acid encoding the polypeptide herein or a portion thereof. Examples of vectors include, but are not limited to, plasmids, viral vectors, non-episomal mammalian vectors and expression vectors, for example, recombinant expression vectors.

The recombinant expression vectors herein can comprise the nucleic acid herein in a form suitable for expression of the nucleic acid in a host cell. The recombinant expression vector includes one or more regulatory sequences, which are screened on the basis of the host cells used for expression, and are operably linked to the pre-expressed nucleic acid sequence. Regulatory sequences include those that direct the constitutive expression of nucleotide sequences in many types of host cells (e.g., an SV40 early gene enhancer, Rous sarcoma virus promoter and cytomegalovirus promoter), those that direct the expression of nucleotide sequences only in certain host cells (e.g., a tissue-specific regulatory sequence, see Voss et al., 1986, Trends Biochem. Sci. 11:287, Maniatis et al., 1987, Science 236:1237, the disclosure of each of which is incorporated by reference herein in its entirety), and those that direct the inducible expression of nucleotide sequences in response to specific treatments or conditions (e.g., a metallothionin promoter in mammalian cells and a tet-responsive promoter and/or streptomycin responsive promoter in both prokaryotic and eukaryotic systems (supra)). It should be understood by one skilled in the art that the design of the expression vector can depend on factors such as the choice of the host cell to be transformed, and the level of expression of the protein desired. The expression vector herein can be introduced into a host cell, thereby producing the protein or peptide, including a fusion protein or peptide, encoded by the nucleic acid described herein.

In another aspect, provided herein is a host cell into which the expression vector herein can be introduced. The host cell may be any prokaryotic or eukaryotic cell. Prokaryotic host cells include Gram-negative or Gram-positive organisms, for example, E. coli or bacilli. More advanced eukaryotic cells include insect cells, yeast cells, and established cell lines of mammalian origin. Examples of appropriate mammalian host cell lines include Chinese hamster ovary (CHO) cells or derivatives thereof such as Veggie CHO and related cell lines which grow in serum-free media (see Rasmussen et al., 1998, Cytotechnology 28:31) or CHO strain DXB-11 which is deficient in DHFR (see Urlaub et al., 1980, Proc. Natl. Acad, Sci. USA 77:4216-20). Additional CHO cell lines include CHO-K1 (ATCC #CCL-61), EM9 (ATCC #CRL-1861) and UV20 (ATCC #CRL-1862). Additional host cells include monkey kidney COS-7 cell lines (ATCC #CRL-1651) (see Gluzman et al., 1981, Cell 23:175), L cells, C127 cells, 3T3 cells (ATCC CCL-163), AM-1/D cells (described in U.S. Patent serial No. 6210924), HeLa cells, BHK (ATCC CRL-10) cell lines, the CV1/EBNA cell line derived from the African green monkey kidney cell line CV1 (ATCC CCL-70) (see McMahan et al., 1991, EMBO J. 10:2821), human embryonic kidney cells such as 293, 293 EBNA or MSR 293, human epidermal A431 cells, human C010205 cells, other transformed primate cell lines, normal diploid cells, cell strains derived from in vitro culture of primary tissue, primary explants, HL-60, U937, HaK or Jurkat cells. Appropriate cloning and expression vectors for bacterial, fungal, yeast, and mammalian cell hosts are described in Pouwels et al. (Cloning Vectors: A Laboratory Manual, Elsevier, 1985).

Vector DNA can be introduced into prokaryotic or eukaryotic cells by traditional transformation or transfection techniques. For stable transfection of mammalian cells, it is known that, depending upon the expression vector and transfection technique used, only a small fraction of cells can integrate the foreign DNA into the genome thereof. In order to identify and screen for these integrants, a gene that encodes a selectable marker (e.g., antibiotic resistance) is generally introduced into a host cell along with the gene of interest. Preferred selectable markers include those which confer resistance to drugs, such as G418, hygromycin and methotrexate. Among other methods, cells stably transfected with the introduced nucleic acid can be identified by drug screening (for example, cells that have integrated the selectable gene will survive, while other cells die).

The transformed cells can be cultured under conditions that promote expression of a polypeptide, and the polypeptide can be recovered by conventional protein purification methods. One such purification method is described in the examples below. Polypeptides contemplated for use herein include substantially homogeneous recombinant mammalian anti-GIPR antibody or FGF21 fusion protein polypeptides, which are substantially free of contaminating endogenous materials.

### Activity of the GIPR antibody

Activity of the GIPR antibody refers to an antibody provided herein having therapeutic biological effects that are shown after specifically binding to a GIPR, inhibiting, or blocking GIP signaling, e.g., in treating non-alcoholic steatohepatitis, obesity, and/or Type 2 diabetes. The term "reducing the biological activity of GIP signaling" or "inhibiting or blocking the biological activity of GIP signaling" refers to binding of the GIPR antibody or its fusion protein with FGF21 to GIPR in vivo, and inhibiting or blocking cellular stress responses downstream of the receptor caused by the GIP. Responses include, but are not limited to, increasing insulin secretion, promoting fat storage, and inhibiting lipolysis. In one embodiment, provided herein is a murine-derived antibody or a humanized antibody capable of specifically binding to a human GIPR. Such antibodies include antagonistic or neutralizing antibodies that can reduce or neutralize GIP signaling.

In one embodiment, the antibody provided herein reduces human GIP signaling with an IC₅₀ value of about 0.01 nM to about 500 nM, about 0.1 nM to about 200 nM, about 0.5 nM to about 200 nM, about 1 nM to about 200 nM, or about 10 nM to about 100 nM. In another embodiment, the antibody provided herein reduces human GIP signaling with an IC₅₀ value of about 1 nM to about 200 nM. In another embodiment, the antibody provided herein reduces human GIP signaling with an IC₅₀ value of about 10 nM to about 100 nM. In another embodiment, the antibody provided herein reduces human GIP signaling with an IC₅₀ value of about 1 nM, about 2 nM, about 5 nM, about 10 nM, about 20 nM, about 30 nM, about 40 nM, about 50 nM, about 60 nM, about 70 nM, about 80 nM, about 90 nM, or about 100 nM.

In one embodiment, the GIPR antibody provided herein, when binding to a human GIPR, has one or more of the properties listed below:
a. providing a K_{d} substantially similar to that of a reference antibody when binding to a human GIPR;
b. providing an IC₅₀ substantially similar to that of the reference antibody when inhibiting GIP activation of a human GIPR; and
c. cross-competing with the reference antibody for binding to a human GIPR.

In another embodiment, the GIPR antibody described herein is an antibody that has one or more of the properties listed below:
a. providing the same or better IC50 as or than a reference GIPR antibody when inhibiting GIP activation of a human GIPR; and
b. the GIPR antibody cross-competing with a reference GIPR antibody for binding to a human GIPR.

Herein, the term "substantially similar" means that the IC₅₀ or K_{d} value of the antibody described herein is comparable to that of the reference antibody, or is about 200%, about 180%, about 160%, about 150%, about 140%, about 120%, about 110%, about 100%, about 99%, about 98%, about 97%, about 95%, about 90%, about 85%, about 80%, about 75%, about 70%, about 65%, or about 50% of that of the reference antibody. In one embodiment, the reference antibody includes, for example, an antibody having a combination of SEQ ID NO: 29 of a light chain and SEQ ID NO: 38 of a heavy chain. In another embodiment, the reference antibody includes the GIPR antibodies L1H1 and L9H1.

### Biological activity of a fusion protein of a GIPR antibody and FGF21

The biological activity of a fusion protein of a GIPR antibody and FGF21 encompasses both the biological activity of the FGF21 and the activity of the GIPR antibody. The activity of the GIPR antibody is as described above. "FGF21 biological activity" refers to the biological activity of a fusion protein of a GIPR antibody and FGF21 in binding in vivo and activating an FGF21 receptor, causing cellular stress responses and thus showing therapeutic effects, for example for non-alcoholic steatohepatitis, obesity, or Type 2 diabetes. Cellular stress responses above include but not limited to, increased secretion of insulins, suppression of secretion of glucagon, inhibition of appetite, weight loss, induction of satiety, inhibition of apoptosis, and induction of pancreatic beta cell proliferation and pancreatic beta cell differentiation. Because of combining the biological activities of FGF21 and a GIPR antibody, the FGF21 fusion protein described herein can be used to treat a variety of diseases and conditions associated with FGFRs and GIPRs. The fusion protein exerts its biological effects by acting on FGF21R and/or GIPR, and therefore, the FGF21 fusion protein described herein can be used to treat subjects with diseases and conditions who respond favorably to "increased FGF21R stimulation" or "reduced GIPR stimulation". These subjects are referred to as subjects who "need FGF21R stimulation treatment" or "need reduced GIPR stimulation". The following subjects are included: subjects with hepatic steatosis (GIPR, see US 2017/0275370A1), non-alcoholic fatty liver disease (FGF21R, see Debra et al., 2016, Hepatobiliary Surg Nutr, 5:515-518; GIPR, see US 2017/0275370 A1), non-alcoholic steatohepatitis (FGF21, see Armstrong et al., 2013, BMJ Open. 3:e003995; GIPR, see US 2017/0275370 A1), non-insulin dependent diabetes, insulin dependent diabetes, stroke (FGF21R, see WO 00/16797), myocardial infarction (FGF21R, see WO 98/08531), obesity (FGF21R, see WO 98/19698; GIPR, see Furija et al., 2008, PLoS ONE 3:e3163; US 2017/0275370 A1), post-operative catabolic alterations (FGF21R, see US 6,006,753), functional dyspepsia and irritable bowel syndrome (FGF21R, see WO 99/64060), and also included are subjects at risk of developing non-insulin dependent diabetes (see WO 00/07617), subjects with impaired glucose tolerance or impaired fasting glucose, subjects whose body weights are 25% higher than the normal weight for the height of the subjects, and subjects with partial pancreatic resection.

In one embodiment, a change in the biological activity of a GIPR antibody or its fusion protein with FGF21 is detected using a reporter gene assay method that quantifies the function of the GIPR antibody or FGF21 fusion protein to inhibit a GIPR in vitro.

### Pharmaceutical composition

In one embodiment, provided herein is a pharmaceutical composition comprising a GIPR antibody provided herein and one or more pharmaceutically acceptable carriers.

In another embodiment, provided herein is a pharmaceutical composition comprising a fusion protein of a GIPR antibody and FGF21 provided herein, and one or more pharmaceutically acceptable carriers.

As used herein, the term "carriers" includes vehicles, pharmaceutical excipients, or stabilizers which are harmless when exposed to cells or mammals at the used dosages and concentrations.

### Treatment method

In one embodiment, provided herein is a method for treating, preventing, or improving non-alcoholic steatohepatitis, comprising administering to a subject a therapeutically effective amount of the GIPR antibody provided herein or a pharmaceutical composition thereof.

In another embodiment, provided herein is a method for treating, preventing, or improving non-alcoholic steatohepatitis, comprising administering to a subject a therapeutically effective amount of a fusion protein of a GIPR antibody and FGF21 provided herein or a pharmaceutical composition thereof.

In another embodiment, provided herein is a method for treating, preventing, or improving obesity, comprising administering to a subject a therapeutically effective amount of the GIPR antibody provided herein or a pharmaceutical composition thereof.

In another embodiment, provided herein is a method for treating, preventing, or improving obesity, comprising administering to a subject a therapeutically effective amount of a fusion protein of a GIPR antibody and FGF21 provided herein or a pharmaceutical composition thereof.

In another embodiment, provided herein is a method for treating, preventing, or improving Type 2 diabetes, comprising administering to a subject a therapeutically effective amount of the GIPR antibody provided herein or a pharmaceutical composition thereof.

In a further embodiment, provided herein is a method for treating, preventing, or improving Type 2 diabetes, comprising administering to a subject a therapeutically effective amount of a fusion protein of a GIPR antibody and FGF21 provided herein or a pharmaceutical composition thereof.

In any of the use provided herein, the pharmaceutical composition is used for intravenous or subcutaneous injection.

Herein, the term "subject" refers to mammals, including humans, and is used interchangeably with the term "patient".

The term "treatment" includes alleviating or preventing at least one symptom or other aspects of a condition, or alleviating the severity of a disease. The GIPR antibody or the fusion protein of the GIPR antibody and FGF21 provided herein can constitute an effective therapeutic agent without the need to produce a complete cure effect or eradicate all symptoms or manifestations of a disease. As recognized in the related art, a drug as a therapeutic agent can be used to reduce the severity of a given disease state, but does not need to eliminate all manifestations of the disease to be considered as an effective therapeutic agent. Similarly, prophylactic administration treatment does not need to be completely effective in preventing the appearance of symptoms to constitute an effective prophylactic agent. It is sufficient to reduce the impact of a disease (e.g., by reducing the number or severity of symptoms thereof, or by enhancing another therapeutic effect, or by producing another effective effect), or to reduce the likelihood of occurrence or aggravation of the disease in a subject. One embodiment herein relates to a method comprising administering the GIPR antibody or the fusion protein of the GIPR antibody and FGF21 to a patient in an amount and time sufficient to induce a sustained improvement of an indicator that reflects the severity of a specific condition above the baseline level.

Pharmaceutical compositions of the GIPR antibody or the fusion protein of the GIPR antibody and FGF21 may be administered by any appropriate technique including but not limited to parenteral, topical or inhalation administration. In the case of injection, a pharmaceutical composition can be administered by, for example, intra-articular, intravenous, intramuscular, intralesional, intraperitoneal or subcutaneous route, as a rapid injection or continuous infusion. For example, local administration at the site of disease or injury may be considered, such as transdermal administration and sustained release administration of implants. Inhalation administration includes, for example, nasal or oral inhalation, sprays, inhalation of antibodies in the form of aerosols, etc. Other options include oral preparations including tablets, syrups or lozenges.

It is advantageous to administer the GIPR antibody or FGF21 fusion protein provided herein in the form of a composition comprising one or more other components, such as a physiologically acceptable carrier, excipient or diluent. The composition may optionally comprise one or more additional physiologically active agents as described below. In various specific embodiments, the composition comprises one, two, three, four, five or six physiologically active agents other than one or more antibodies (such as a murine-derived antibody or a humanized antibody) or FGF21 fusion proteins provided herein.

In one embodiment, the pharmaceutical composition comprises the fusion protein of a murine-derived antibody or a humanized antibody or an FGF21 fusion protein provided herein, and one or more substances selected from: buffers with pH suitable for the antibody or FGF21 fusion protein, antioxidants such as ascorbic acid, low molecular weight polypeptides (such as polypeptides comprising less than 10 amino acids), proteins, amino acids, carbohydrates such as dextrin, complexes such as EDTA, glutathione, stabilizers and excipients. According to appropriate industry standards, preservatives may also be added. The composition can be formulated into a freeze-dried powder using a suitable excipient solution as a diluent. Appropriate components are non-toxic to recipients at the dosage and concentration used. Further examples of components that can be used in pharmaceutical prescriptions are described in Remington's Pharmaceutical Sciences, 16th edition (1980) and 20th edition (2000). Mack Publishing Company provides a kit for use by medical practitioners, which includes one or more antibodies or FGF21 fusion proteins provided herein, and tags or other instructions for treating any of the diseases discussed herein. In one embodiment, the kit includes a sterile preparation of one or more antibodies or FGF21 fusion proteins in one or more tube-type bottles in the form of the above-mentioned composition.

The dosage and frequency of administration can vary based on the following factors: the route of administration, the specific antibodies or FGF21 fusion proteins used, the nature and severity of the disease to be treated, whether the symptoms are acute or chronic, and the size and overall symptoms of the patient. The appropriate dose can be determined by methods well known in the art, for example, dose scaling studies in clinical trials.

The antibody or FGF21 fusion protein provided herein can be administered, for example, once or several times at regular intervals over a period of time. In a specific embodiment, a murine-derived or humanized antibody or FGF21 fusion protein is administered once in at least one month or longer, for example, one, two, or three months or even indefinitely. For the treatment of chronic symptoms, long-term treatment is usually the most effective. However, for the treatment of acute symptoms, short-term administration, for example, from one week to six weeks is sufficient. Generally, human antibodies are administered until a patient shows a medically relevant improvement of the selected sign or an indicator above the baseline level.

One example of a treatment regimen provided herein includes the treatment of symptoms caused by non-alcoholic steatohepatitis, obesity, Type 2 diabetes or the like with subcutaneous injection of the antibody or FGF21 fusion protein at an appropriate dose once a week or less frequently. The antibody or FGF21 fusion protein may be continuously administered weekly or monthly until a desired result is achieved, for example resolution of the patient's symptoms. The treatment can be renewed as needed, or, alternatively, a maintenance dose can be administered.

The patient can be monitored for liver fat fraction (HFF), liver injury marker alanine aminotransferase (ALT), body weight, blood glucose concentration to detect any change in these parameters, before, during and/or after the treatment with an antibody or FGF21 fusion protein, for example a human antibody or FGF21 fusion protein. For certain conditions, changes in HFF, ALT, and blood glucose can vary with factors such as disease progression. The changes in levels thereof can be determined using known techniques.

Specific embodiments of the methods and compositions herein involve the use of, for example, antibodies or FGF21 fusion proteins and one or more GIP antagonists, two or more antibodies or FGF21 fusion proteins provided herein, or antibodies or FGF21 fusion proteins of the present disclosure and one or more other GIP antagonists. In a further embodiment, an antibody or an FGF21 fusion protein is administered alone or in combination with other agents for treating distressing symptoms of the patient. Examples of these agents include proteins and non-protein drugs. When multiple drugs are administered in combination, the dosage thereof should be adjusted accordingly as is well known in the art. "Combined administration" combination therapy is not limited to simultaneous administration, and also includes a treatment regimen in which an antigen and a protein are administered at least once during a course of treatment involving the administration of at least one other therapeutic agent to a patient.

In another aspect, provided herein is a method for preparing a medicament for treating non-alcoholic steatohepatitis, obesity, and Type 2 diabetes and related conditions, wherein the medicament comprises a mixture of the antibody or FGF21 fusion protein provided herein and a pharmaceutically acceptable excipient for the treatment of conditions associated with the diseases above. The method for preparing the medicament is as described above.

Further provided herein are compositions, kits and methods related to an antibody or an FGF21 fusion protein that can specifically bind to a human GIPR. Also provided are nucleic acid molecules, and derivatives and fragments thereof, comprising a polynucleotide encoding all or a portion of a polypeptide that binds to a GIPR, for example a nucleic acid encoding all or a portion of an anti-GIPR antibody, antibody fragment, antibody derivative or an FGF21 fusion protein. Further provided herein are vectors and plasmids comprising such nucleic acids, and cells and cell lines comprising such nucleic acids and/or the vectors and plasmids. Provided methods include, for example, a method for preparing, identifying, or isolating an antibody or FGF21 fusion protein that binds to a human GIPR, e.g., the anti-GIPR antibody or FGF21 fusion protein, a method for determining whether the antibody or FGF21 fusion protein binds to a GIPR, and a method for administering the antibody or FGF21 fusion protein that binds to a GIPR to an animal model.

The following specific examples are used to further illustrate the technical solutions herein.

Herein, unless otherwise specified, the raw materials, equipment and the like used can all be purchased from the market or are commonly used in the art. The methods in the following examples, unless otherwise specified, are conventional methods in the art.

### 1. Preparation of antigens for immunization

CHO-DHFR-cells were inoculated into a 6-well plate. After 24 hours (hrs) of culture, cells in the 6-well plate were transfected with the pTM15 plasmid cloned with hGIPR gene (see SEQ ID NO: 114 for the nucleotide sequence and SEQ ID NO: 113 for the amino acid sequence). Transfection was performed according to the transfection conditions recommended by Invitrogen for Lipofectamine 2000. After 48 hrs, the culture solution was changed to a complete medium containing 300 µg/mL hygromycin, and the medium was changed every 3 days (d) apart. After about two weeks of culture, stable clones appeared. Cell colonies were dispersed by digestion, and the cells were passaged, and continued to be cultivated until growing to 100% confluence. The constructed stable cell strains were separately detected by FACS using V5-tag antibody (Life Technologies), and the cell populations after pressure screening were identified according to the FACS detection results. After screening, a large amount of hGIPRs were expressed on CHO-DHFR-hGIPR cell membranes. Finally, after subcloning and further identification, 3 strains of GIPR cells were selected as highly-expressed stable cell strains. These cell strains highly expressing hGIPRs can be used as immunogens for preparing antibodies (see Example 2). In addition, in certain embodiments, a fusion protein of the hGIPR extracellular region and hlgG Fc can also be used as an immunogen for preparing antibodies, and the preparing method thereof was as follows: The gene sequence of the fusion protein of the hGIPR extracellular region, hlgG2 Fc and peptide linker (Linker) was subcloned into the pTM5 plasmid. High-level transient expression was performed by suspension-cultured HEK293 cells, the cell supernatant was obtained, and then purified by affinity chromatography to obtain the hGIPR extracellular region fusion protein.

### 2. Preparation of antibodies

Antibodies against hGIPRs can be produced using any of the following immunogens. For example, in certain embodiments, whole cells expressing hGIPRs were used as immunogens to produce antibodies against hGIPRs. In addition, in certain embodiments, fusion proteins comprising an hFc and the amino acid sequence of the N-terminal domain of an hGIPR were used as immunogens to produce antibodies against hGIPRs. An immunogen and an aluminum hydroxide adjuvant were mixed evenly, and then injected subcutaneously into BALB/c mice (6-8 weeks old) and the mice were subjected to booster immunization weekly thereafter. After a total of 6 immunizations, blood was collected by tail snipping. The serum was isolated by centrifugation and serum titers were determined by FACS. When appropriate antibody titers were reached, mice were sacrificed by cervical dislocation and spleen cells were obtained aseptically. SP2/0 cells in logarithmic phase were collected, and centrifuged. The cell precipitate was resuspended in a serum-free culture medium, then centrifuged and resuspended for a second time, and counted. Spleen cells and SP2/0 cells were mixed to ensure that the number of SP2/0 cells is close to that of spleen cells. After mixing, 3 rounds of washing-centrifugation were performed. After the cell precipitate from the last centrifugation was detached, pre-warmed PEG-1500 was added dropwise. The mixture was pipetted up and down, and then 30 mL of a pre-warmed serum-free culture medium was added slowly to terminate the PEG fusion. After centrifugation again, the cell precipitate was detached, to which a fusion culture medium was added. Spleen cells and feeder layer cells were plated into a 96-well plate, with 100 µL of culture medium added per well. The fused hybridoma cells and feeder layer cells were co-cultured in the 96-well plate, and subjected to HAT (hypoxanthine, methotrexate and thymidine) screening to remove non-fused cells. After 10 d, the hybridoma cell supernatants in the culture plate were harvested for ELISA detection.

### 3. Antibody screening by ELISA

CHO-DHFR-hGIPR cells overexpressing hGIPRs and CHO-DHFR-cells not expressing hGIPRs were inoculated separately into 96-well plates. After the cells have grown to 90% confluence, the cell culture supernatant was removed. The cells were washed twice with PBS, fixed at 4°C by adding 100% methanol, and then treated for 20 min at room temperature by adding 100 µL of freshly prepared 0.6% H₂O₂-PBS, and washed twice with PBS. After blocking with 1% BSA (dissolved in PBS), the hybridoma cell supernatant was added, and the mixture was incubated at 4°C for 90 min. After several washes, 100 µL of diluted goat anti-murine Fc-HRP secondary antibody (Sigma-Aldrich) was added to each well, and the mixture was incubated at 37°C for 30 min. After 5 washes, 100 µL of a TMB chromogenic substrate was added to each well, reaction was performed at 37°C for 15 min, 50 µL of 2 M H₂SO₄ was added for terminating color development, and OD 450 values were read. In addition, in certain embodiments, fusion proteins comprising an hFc and the amino acid sequence of the N-terminal domain of an hGIPR were used as coating antigens to coat the 96-well plate. After blocking with 1% BSA (dissolved in PBS), the hybridoma cell supernatant was added, and the mixture was incubated at 4°C for 90 min. The subsequent steps are the same as the above-mentioned ELISA method, for screening anti-hGIPR monoclonal antibodies. The positive control was the serum of immunized mice; and the negative control was the cell culture medium supernatant. After preliminary detection by ELISA, several positive hybridoma cell strains secreting anti-hGIPR antibodies were screened out. These hybridoma strains secreting anti-hGIPR antibodies were selected and cloned to obtain cell strains capable of stably secreting anti-hGIPR antibodies. Finally, the positive hybridoma cell supernatant was selected for verification by FACS (see Example 10).

### 4. Cloning and subcloning of antibody genes

Hybridoma cells secreting antibodies were collected, and according to the manufacturer protocol of QIAGEN mRNA extraction kit, the mRNA of the hybridoma cells was extracted. Then the extracted mRNA was transcribed reversely into cDNA. The reverse transcription primers were specific primers for the light and heavy chain constant regions of a mouse, with the heavy chain reverse transcription primer being (5'-TTTGGRGGGAAGATGAAGAC-3'), and the light chain reverse transcription primers being (5'-TTAACACTCTCCCCTGTTGAA-3') and (5'-TTAACACTCATTCCTGTTGAA-3'). The reaction conditions of RT-PCR were: 25°C for 5 min; 50°C for 60 min; and 70°C for 15 min. The reversely-transcribed cDNA was diluted to 500 µL with 0.1 mM TE, added to an ultrafiltration centrifuge tube (Amicon Ultra-0.5), and centrifuged at 2000 g for 10 min; the filtrate was discarded, and 500 µL of 0.1 mM TE was added and centrifuged at 2000 g for 10 min; the filtrate was discarded, and the preparation tube was inverted into a new centrifuge tube and centrifuged at 2000 g for 10 min to obtain purified cDNA; 10 µL of the purified cDNA was taken as a template, 4 µL of 5×tailing buffer (Promega), 4 µL of dATP (1 mM) and 10 U terminal transferase (Promega) were added and mixed uniformly, and the mixture was incubated at 37°C for 5 min and then incubated at 65°C for 5 min; and then the cDNA with PolyA tail was used as a template to amplify the genes of the light and heavy chain variable regions of the antibody by PCR. The upstream primers were all OligodT, and the downstream primers of the heavy chain were (5'-TGGACAGGGATCCAGAGTTCC-3') and (5'-TGGACAGGGCTCCATAGTTCC-3'), and the downstream primer of the light chain was (5'-ACTCGTCCTTGGTCAACGTG-3'). The reaction conditions of PCR were: 95°C for 5 min; 95°C for 30 s, 56°C for 30 s, 72°C for 1 min, 40 cycles; and 72°C for 7 min. The PCR product was ligated to PMD 18-T vector (Takara Bio) and then sequenced. PCR primers were designed on the basis of the sequenced DNA sequences of the antibody, and thus the complete light chain and heavy chain signal peptides and variable domains and mouse IgG1 constant region were ligated to expression vector pTM5.

### 5. Antibody humanization and optimization

First, based on the murine-derived antibody light, heavy chain variable region sequences obtained from the screening, human-derived antibody germline gene sequences (Ig Germline Gene Sequence) homologous to the murine-derived antibody variable region sequences obtained from the screening were searched using the NCBI database, and the human-derived gene sequences having the highest homology except CDR sequences were used as template sequences for CDR grafting to obtain humanized antibody variable region sequences. Genes of the light, heavy chains of a humanized antibody were synthesized, and linked with human IgG2 or IgG4 constant region sequences to obtain a complete recombinant humanized antibody sequence. The expression of the recombinant antibodies was performed according to Example 8, and their affinity for GIPRs was verified by the FACS technique in step 12 to select the antibodies that displayed the best affinity. Finally, the variable region sequence of the humanized antibody was modified by site-directed mutagenesis to further increase the affinity of the antibody for GIPRs.

### 6. Genetic cloning and subcloning of humanized hGIPR antibodies

Outsourced synthesis of optimized humanized antibody heavy and light chain variable region sequences was carried out. During synthesis, a restriction enzyme site Nhe1 was introduced into the 5' end of the heavy chain variable region, and a restriction enzyme site Sal1 was introduced into the 3' end, so that the complete heavy chain variable region sequence was ligated to the expression vector pTM5 into which the heavy chain constant region had been incorporated. Similarly, during synthesis, a restriction enzyme site Nhe1 was introduced into the 5' end of the light chain variable region, and a restriction enzyme site Bsiw1 was introduced into the 3' end, so that the complete light chain variable region sequence was ligated to the expression vector pTM5 into which the light chain constant region had been incorporated.

### 7. Construction of a fusion protein of a humanized hGIPR antibody and FGF21

The optimized humanized antibody was fused to FGF21 and its derivative sequence at the C-terminus of the heavy chain to form an FGF21 fusion protein (e.g., SEQ ID NOs: 104-106, SEQ ID NOs: 119-121, SEQ ID NO: 123 to SEQ ID NO: 125). The two sequences were linked by a peptide linker sequence (Linker). The nucleotide sequence of Linker-FGF21 was synthesized by GenScript Biotech Co., Ltd. Using the synthetic gene as a template, the sequence of the "Linker-FGF21" part was amplified by PCR. In addition, using the nucleotide sequence of the humanized antibody as a template, the sequence of the signal peptide-antibody part of the fusion protein sequence was amplified. Then through overlapping PCR, the part "Linker-FGF21" of the nucleic acid sequence of the fusion protein is connected with the antibody part, introducing two restriction enzyme sites Nhe1 and Not1 to both ends of the primers, and thus complete fusion protein sequence and the expression vector pTM5 are ligated together.

### 8. Transient expressions of an hGIPR antibody, an FGF21 fusion protein and FC-FGF21 (RGE)

Suspension-cultured HEK293 or CHO expression cell strains at 5 × 10⁵/mL were inoculated into a spinner flask. After 24 hrs of rotary culture at 37°C and 5% CO₂, when the density reached 1 × 10⁶/mL, the cells were used for transfection. Polyethylenimine (PEI) was used as a transfection medium during the transfection process, and mixed with DNA. The mixture of the two materials was added to the cell culture after 15 min of standing incubation. After receiving the mixture of PEI and DNA, the cells were cultured under rotation for 24 hrs at 37°C and 5% CO₂, and then tryptone was added to the cell culture solution as an additive required for expression. Finally, after expression was complete (over 96 hrs), cell supernatant was collected for antibody purification and isolation.

### 9. Purification and isolation of an hGIPR antibody, an FGF21 fusion protein and FC-FGF21 (RGE)

The collected cell supernatant in Example 8 was centrifuged at high speed (8000 rpm) to remove cells and cell debris, and then filtered and clarified with a 0.22 µm filter membrane. The clarified supernatant was used for purification. The purification process was completed by a chromatograph. The supernatant first flowed through a protein A/G affinity chromatography column. The antibody contained in the supernatant bound to the ligand of the protein A/G affinity chromatography column during this time, and then retained in the column. The chromatography column was then rinsed with an elution buffer at a low pH (at or less than 3.0) to dissociate the antibody bound to the chromatography column. The collected antibody eluate was rapidly neutralized with 1 M Tris-HCl. The resulting antibody eluate was dialyzed and then replaced with a PBS or other buffer system.

### 10. Detection of the construction of stable cell strains expressing the β-Klotho/FGFR1c receptor complex

After inoculating CHO-DHFR cells (commercially available) into a 6-well plate, and culturing for 24 h, cells in the 6-well plate were transfected with the plasmid containing the V5-hKLB gene and the plasmid containing Flag-FGFR1c, wherein the culture medium was changed before transfection, and the transfection was performed according to the transfection conditions recommended by Invitrogen for Lipofectamine 2000. 48 hours after transfection, the culture medium was replaced by the complete medium containing 10 nM methotrexate (MTX). The medium is changed every 3 days apart for about two weeks, until stable clones appear. The cell colonies were dispersed by digestion. After cells grow to about 50% confluence, gradually increasing concentrations of MTX (up to a concentration of 10 µM MTX) were added for pressure screening. The constructed stable cell strains were tested respectively by FACS using the antibodies against the KLB protein N'-terminal V5-tag (FITC-anti-V5, Invitrogen; Flag-anti-V5, Invitrogen) to identify cell populations after pressure screening. After screening with 10 uM MTX, a high level of hKLB was expressed on the CHO-DHFR-V5-hKLB cell membranes. Finally, 2 stable cell strains with high KLB expression were obtained through subcloning and identification.

### 11. A reporter gene experiment detecting the biological activity of the hGIPR antibody/FGF21 fusion protein in activating the hFGFR1c/hKLB signaling pathway in vitro

CHO-DHFR-cells co-expressing hFGFR1c-β-KLB-SRE-Luciferase were inoculated into a 96-well cell culture plate at 20,000 cells per well, and cultured overnight at 37°C and 5% CO₂. The next day, the culture supernatant was removed. The cell surfaces were washed twice with a serum-free medium and residual liquid was removed, then 100 µL of purified antibodies or FGF21 diluted with the serum-free medium was added, and the mixture was incubated at 37°C for 4 hours. After the stimulation, 100 µL of Bright Glo chemiluminescent substrate (Promega) was added, and finally the cell lysate was transferred to a white 96-well plate, and the relative fluorescence intensity was read on the SpectraMax L microplate reader (Molecular Devices) (see FIG. 1 for the results).

### 12. A reporter gene experiment detecting the biological activity of the hGIPR antibody/FGF21 fusion proteins V1W1 and V1W2 in antagonizing a GIPR in vitro

CHO-DHFR-cells co-expressing hGIPR-CRE-Luciferase were inoculated into a 96-well cell culture plate at 30000 cells per well, and cultured overnight in an incubator at 37°C and 5% CO₂. The next day, the cell supernatant was removed. The cell surfaces were washed twice with a serum-free medium, and the residual liquid was removed, and then 50 µL of GIP diluted with the serum-free medium and 50 µL of hGIPR antibodies or hGIPR antibody/FGF21 fusion proteins gradient-diluted with the serum-free medium were added to each well respectively, and the mixture was incubated at 37°C for 4 hours. After the stimulation, 100 µL of Bright Glo chemiluminescent substrate (Promega) was added, and finally the cell lysate was transferred to a white 96-well plate, and the relative fluorescence intensity was read on the SpectraMax L microplate reader (Molecular Devices) (see FIG. 2 for the results).

### 13. A reporter gene experiment detecting the biological activity of the hGIPR antibody/FGF21 fusion proteins V1W1 and V1W2 in activating hβ-Klotho/hFGF1Rc in vitro

CHO-DHFR-cells co-expressing hFGFR1c-β-KLB-SRE-Luciferase or mFGFR1c-β-KLB-SRE-Luciferase were inoculated into a 96-well cell culture plate at 20,000 cells per well, and cultured overnight at 37°C. The next day, the culture supernatant was removed. The cell surfaces were washed twice with a serum-free medium and residual liquid was removed, then 100 µL of purified antibodies or FGF21 diluted with the serum-free medium was added, and the mixture was incubated at 37°C for 4 hours. After the stimulation, 100 µL of Bright Glo chemiluminescent substrate (Promega) was added, and finally the cell lysate was transferred to a white 96-well plate, and the relative fluorescence intensity was read on the SpectraMax L microplate reader (Molecular Devices) (see FIG. 3 and FIG. 8 for the results).

### 14. The evaluation of the efficacy of the hGIPR antibody/FGF21 fusion protein in vivo in high-fat feed-induced C57BL/6 obese mice in a pharmacodynamic experiment

A obesity model was established by induction with a 60% high-fat feed in C57BL/6 mice (DIO mice). After the mice were purchased, they were fed with a normal diet for a week, and then a certain number of mice were randomly selected as normal controls and fed with the common mouse feed, and the remaining animals were fed with the high-fat feed. The mice were fed continuously for 8 weeks (wk), and their body weights and food intakes were measured once a week. Subsequently, the mice in the high-fat feed group were randomly divided by body weight into the V1W1-FGF21 (SEQ: 89) group (1 mg/kg), V1W1-FGF21 (SEQ: 89) group (3 mg/kg), V1W1-FGF21 (SEQ: 89) group (10 mg/kg), V1W2-FGF21 (SEQ: 89) group (1 mg/kg), V1W2-FGF21 (SEQ: 89) group (3 mg/kg), and V1W2-FGF21 (SEQ: 89) group (10 mg/kg), a positive drug control group, and a model group. Subcutaneous injection of drugs was performed once every three days, for a total duration of 4 wks, the normal control group was not dosed, and the model group was dosed with an equal amount of a blank preparation. The data of body weights, food intakes, and behavioral observations of mice were collected during the experimental period. Fasting blood glucose levels were measured on days 0, 7, 14, 21, and 28 of the experiment. The oral glucose tolerance test (OGTT) was performed on days 14 and 28 of the experiment. The mice were fasted (free access to water) for 12 hrs before the last day of the experiment, blood was collected from orbits of the animals to isolate sera and the euthanasia was performed, thereby detecting serum ALT, AST, HDL-C, LDL-C, TC and TG (see FIG. 4, FIG. 5, FIG. 6 and FIG. 7 for the results).

### 15. The evaluation of the efficacy of the hGIPR antibody/FGF21 fusion protein in vivo in C57BL/6 mice with non-alcoholic steatohepatitis induced by choline-deficient L-amino-acid-defined high-fat feed in a pharmacodynamic experiment

A non-alcoholic steatohepatitis model was established by induction with the choline-deficient L-amino-acid-defined high-fat feed in C57BL/6 mice. After the mice were purchased, they were fed with a normal diet for a week, and then a certain number of mice were randomly selected as normal controls and fed with the common mouse feed, and the remaining animals were fed with the choline-deficient L-amino-acid-defined high-fat feed. Mice were continuously fed for 6 weeks, and the mice in the choline-deficient L-amino-acid-defined high-fat feed group were randomly divided by TC, TG contents in the serum into the V1W2-FGF21 (SEQ: 118) group (1 mg/kg), V1W2-FGF21 (SEQ: 118) group (3 mg/kg), and V1W2-FGF21 (SEQ: 118) group (10 mg/kg), a positive drug control group, and a model group. Subcutaneous injection of drugs was performed once every three days, for a total duration of 8 wks, the normal control group was not dosed, and the model group was dosed with an equal amount of a blank preparation. The data of body weights, food intakes, and behavioral observations of mice were collected during the experimental period. At week 8 of the experiment, blood was collected from mice and the serum was isolated for the detection of ALT, AST, TG and TC. The livers of mice were collected and weighed. 95-105 mg of liver tissue was weighed for the detection of TC and TG, and the liver from the same lobe was taken and fixed in 4% paraformaldehyde for HE and Sirius Red staining (see FIG. 9 for the results).

The above examples are provided to provide one of ordinary skill in the art with sufficient disclosure and illustration of how to make and use the claimed embodiments, and are not meant to limit the scope of the disclosure herein. For one skilled in the art, obvious modifications all fall within the scope of the claims herein. All publications, patents and patent applications cited in this description are incorporated herein by reference as if each publication, patent or patent application is specifically and individually incorporated herein by reference.

## Claims

1. An antibody capable of specifically binding to a human GIPR, wherein the antibody comprises one, two, three, four, five, or six amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11;
b. light chain CDR2 amino acid sequences: YAS, SEQ ID NO: 2;
c. light chain CDR3 amino acid sequences: SEQ ID NO: 3 and SEQ ID NO: 4;
d. heavy chain CDR1 amino acid sequence: SEQ ID NO: 12;
e. heavy chain CDR2 amino acid sequence: SEQ ID NO: 13; and
f. heavy chain CDR3 amino acid sequence: SEQ ID NO: 14.

2. The antibody of claim 1, wherein the antibody comprises one or two amino acid sequences, wherein each amino acid sequence is independently selected from the amino acid sequences listed below:
a. light chain CDR1 amino acid sequences: SEQ ID NO: 1, SEQ ID NO: 5, SEQ ID NO: 6, SEQ ID NO: 7, SEQ ID NO: 8, SEQ ID NO: 9, SEQ ID NO: 10 and SEQ ID NO: 11; and
b. heavy chain CDR1 amino acid sequence: SEQ ID NO: 12.

3. The antibody of claim 1 or 2, wherein the antibody comprises or further comprises one or two amino acid sequences, wherein each amino acid sequence is independently selected from the amino acid sequences listed below:
a. light chain CDR2 amino acid sequences: YAS, SEQ ID NO: 2; and
b. heavy chain CDR2 amino acid sequence: SEQ ID NO: 13.

4. The antibody of any one of claims 1 to 3, wherein the antibody comprises or further comprises one or two amino acid sequences, wherein each amino acid sequence is independently selected from the amino acid sequences listed below:
a. light chain CDR3 amino acid sequences: SEQ ID NO: 3 and SEQ ID NO: 4; and
b. heavy chain CDR3 amino acid sequence: SEQ ID NO: 14.

5. The antibody of any one of claims 1 to 4, wherein the antibody comprises or further comprises one or two amino acid sequences, wherein each amino acid sequence is independently selected from the amino acid sequences listed below: SEQ ID NO: 1, YAS, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

6. The antibody of any one of claims 1 to 5, wherein the antibody comprises or further comprises one or two amino acid sequences, wherein each amino acid sequence is independently selected from the amino acid sequences listed below: SEQ ID NO: 12, SEQ ID NO: 13 and SEQ ID NO: 14.

7. The antibody of any one of claims 1 to 6, wherein the antibody comprises or further comprises a combination of light and heavy chain CDR1 amino acid sequences independently selected from the list below: SEQ ID NO: 1 and SEQ ID NO: 12, SEQ ID NO: 5 and SEQ ID NO: 12, SEQ ID NO: 6 and SEQ ID NO: 12, SEQ ID NO: 7 and SEQ ID NO: 12, SEQ ID NO: 8 and SEQ ID NO: 12, SEQ ID NO: 9 and SEQ ID NO: 12, SEQ ID NO: 10 and SEQ ID NO: 12, and SEQ ID NO: 11 and SEQ ID NO: 12.

8. The antibody of any one of claims 1 to 7, wherein the antibody comprises or further comprises a combination of light and heavy chain CDR2 amino acid sequences independently selected from the list below: SEQ ID NO: 2 and SEQ ID NO: 13, and YAS and SEQ ID NO: 13.

9. The antibody of any one of claims 1 to 8, wherein the antibody comprises or further comprises a combination of light and heavy chain CDR3 amino acid sequences independently selected from the list below: SEQ ID NO: 3 and SEQ ID NO: 14, and SEQ ID NO: 4 and SEQ ID NO: 14.

10. The antibody of any one of claims 1 to 9, wherein the antibody comprises
(a)
light chain CDR1 amino acid sequence: SEQ ID NO: 1;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(b)
light chain CDR1 amino acid sequence: SEQ ID NO: 5;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(c)
light chain CDR1 amino acid sequence: SEQ ID NO: 6;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(d)
light chain CDR1 amino acid sequence: SEQ ID NO: 1;
light chain CDR2 amino acid sequences: YAS, SEQ ID NO: 2;
light chain CDR3 amino acid sequence: SEQ ID NO: 4;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(e)
light chain CDR1 amino acid sequence: SEQ ID NO: 7;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(f)
light chain CDR1 amino acid sequence: SEQ ID NO: 8;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(g)
light chain CDR1 amino acid sequence: SEQ ID NO: 9;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(h)
light chain CDR1 amino acid sequence: SEQ ID NO: 10;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14;
(i)
light chain CDR1 amino acid sequence: SEQ ID NO: 11;
light chain CDR2 amino acid sequences: YAS, SEQ ID NO: 2;
light chain CDR3 amino acid sequence: SEQ ID NO: 3;
heavy chain CDR1 amino acid sequence: SEQ ID NO: 12;
heavy chain CDR2 amino acid sequence: SEQ ID NO: 13; and
heavy chain CDR3 amino acid sequence: SEQ ID NO: 14.

11. The antibody of any one of claims 1 to 10, wherein the antibody comprises one or two amino acid sequences, wherein each amino acid sequence is independently selected from the amino acid sequences listed below:
a. light chain variable domain amino acid sequences: SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36 and SEQ ID NO: 37; and an amino acid sequence that is at least 80%, at least 85%, at least 90%, or at least 95% identical to any of these sequences; and
b. heavy chain variable domain amino acid sequence: SEQ ID NO: 38; and an amino acid sequence that is at least 80%, at least 85%, at least 90%, or at least 95% identical thereto.

12. The antibody of any one of claims 1 to 11, wherein the polynucleotide coding sequence of the antibody comprises one or two polynucleotide sequences, wherein each polynucleotide sequence is independently selected from the polynucleotide sequences listed below:
a. light chain variable domain polynucleotide coding sequences: SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 and SEQ ID NO: 47; and a polynucleotide sequence that is at least 80%, at least 85%, at least 90%, or at least 95% identical to any of these sequences; and
b. heavy chain variable domain polynucleotide coding sequence: SEQ ID NO: 48; and a polynucleotide sequence that is at least 80%, at least 85%, at least 90%, or at least 95% identical thereto.

13. The antibody of any one of claims 1 to 12, wherein the antibody comprises or further comprises an amino acid sequence independently selected from the list below: SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36 and SEQ ID NO: 37.

14. The antibody of any one of claims 1 to 13, wherein the antibody comprises or further comprises an amino acid sequence independently selected from the list below: SEQ ID NO: 38.

15. The antibody of any one of claims 1 to 14, wherein the antibody comprises a combination of light and heavy chain variable region amino acid sequences independently selected from the list below: SEQ ID NO: 29 and SEQ ID NO: 38, SEQ ID NO: 30 and SEQ ID NO: 38, SEQ ID NO: 31 and SEQ ID NO: 38, SEQ ID NO: 32 and SEQ ID NO: 38, SEQ ID NO: 33 and SEQ ID NO: 38, SEQ ID NO: 34 and SEQ ID NO: 38, SEQ ID NO: 35 and SEQ ID NO: 38, SEQ ID NO: 36 and SEQ ID NO: 38, and SEQ ID NO: 37 and SEQ ID NO: 38.

16. The antibody of any one of claims 15, wherein the antibody comprises or further comprises an amino acid sequence independently selected from the list below: SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37 and SEQ ID NO: 38.

17. The antibody of any one of claim 16, wherein the antibody comprises a combination of light and heavy chain variable region amino acid sequences independently selected from the list below: SEQ ID NO: 29 and SEQ ID NO: 38, SEQ ID NO: 30 and SEQ ID NO: 38, SEQ ID NO: 34 and SEQ ID NO: 38, SEQ ID NO: 35 and SEQ ID NO: 38, SEQ ID NO: 36 and SEQ ID NO: 38, and SEQ ID NO: 37 and SEQ ID NO: 38.

18. The antibody of claim 16, wherein the antibody comprises the amino acid sequence SEQ ID NO: 29 or SEQ ID NO: 38.

19. The antibody of claim 16, wherein the antibody comprises a combination of the amino acid sequences of SEQ ID NO: 29 and SEQ ID NO: 38.

20. The antibody of any one of claims 1 to 19, wherein the antibody further comprises one or two amino acid sequences, wherein each amino acid sequence is independently selected from the amino acid sequences listed below:
a. light chain constant region amino acid sequence: SEQ ID NO: 49; and
b. heavy chain constant region amino acid sequences: SEQ ID NO: 50, SEQ ID NO: 51.

21. The antibody of any one of claims 1 to 20, wherein the antibody is a murine-derived GIPR antibody or a humanized GIPR antibody.

22. The antibody of any one of claims 1 to 21, wherein the antibody is a GIPR monoclonal antibody.

23. The antibody of any one of claims 1 to 22, wherein the antibody is a monoclonal antibody comprising a combination of amino acid sequences selected from the list below: SEQ ID NO: 29 and SEQ ID NO: 38, SEQ ID NO: 30 and SEQ ID NO: 38, SEQ ID NO: 31 and SEQ ID NO: 38, SEQ ID NO: 32 and SEQ ID NO: 38, SEQ ID NO: 33 and SEQ ID NO: 38, SEQ ID NO: 34 and SEQ ID NO: 38, SEQ ID NO: 35 and SEQ ID NO: 38, SEQ ID NO: 36 and SEQ ID NO: 38, and SEQ ID NO: 37 and SEQ ID NO: 38.

24. The antibody of any one of claims 1 to 23, wherein the antibody has one or more of the properties listed below:
a. providing the same or better Kd as or than a reference GIPR antibody when binding to a human GIPR;
b. providing the same or better IC50 as or than a reference GIPR antibody when inhibiting GIP activation of a human GIPR; and
c. the GIPR antibody cross-competing with a reference GIPR antibody for binding to a human GIPR.

25. The antibody of claim 24, wherein the antibody cross-competes with the reference GIPR antibody for binding to a human GIPR.

26. The antibody of claim 24 or 25, wherein the reference GIPR antibody comprises the antibody of any one of claims 1 to 24.

27. The antibody of claim 26, wherein the reference GIPR antibody comprises a combination of the light chain variable domain amino acid sequence SEQ ID NO: 29 and the heavy chain variable domain amino acid sequence SEQ ID NO: 38.

28. The antibody of any one of claims 1 to 27, wherein: the antibody is a murine-derived antibody, a human antibody, a humanized antibody, a chimeric antibody, a monoclonal antibody, a polyclonal antibody, a recombinant antibody, an antigen-binding antibody fragment, a single-chain antibody, a diabody, a triabody, a tetrabody, an Fab fragment, an F(ab')x fragment, a domain antibody, an IgD antibody, an IgE antibody, an IgM antibody, an IgG1 antibody, an IgG2 antibody, an IgG3 antibody, or an IgG4 antibody.

29. The antibody of any one of claims 1 to 28, wherein the antibody has an IC₅₀ value of approximately 1 nM to 200 nM or 1 nM to 100 nM in reducing human GIP signaling.

30. An FGF21 fusion protein, wherein the structure of the fusion protein is **characterized in that**: the fusion protein comprises a GIPR antibody and one, two, three, four, five, six, seven, or eight FGF21 fragments; in the fusion protein, the amino terminus of an FGF21 fragment is linked to the carboxy terminus of a GIPR antibody light or heavy chain via a peptide linker sequence (Linker), wherein the FGF21 fragment comprises an amino acid sequence independently selected from one of the following sequences: SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 115, SEQ ID NO: 116, SEQ ID NO: 117, SEQ ID NO: 118 and SEQ ID NO: 122.

31. An FGF21 fusion protein, wherein the structure of the fusion protein is **characterized in that**: the fusion protein comprises a GIPR antibody of any one of claims 1 to 29 and one, two, three, four, five, six, seven, or eight FGF21 fragments; in the fusion protein, the amino terminus of an FGF21 fragment is linked to the carboxy terminus of a GIPR antibody light or heavy chain via a peptide linker sequence (Linker).

32. The fusion protein of claim 30 or 31, wherein the fusion protein comprises a GIPR antibody and one, two, three, or four FGF21 fragments; in the fusion protein, the amino terminus of an FGF21 fragment is linked to the carboxy terminus of a GIPR antibody light or heavy chain via a peptide linker sequence (Linker).

33. The fusion protein of claims 30 to 32, wherein the fusion protein comprises a GIPR antibody and one, or two FGF21 fragments; in the fusion protein, the amino terminus of an FGF21 fragment is linked to the carboxy terminus of a GIPR antibody light or heavy chain via a peptide linker sequence (Linker).

34. The fusion protein of claims 30 to 33, wherein the GIPR antibody, FGF21 fragment, and peptide linker sequence (Linker) are fused to form the fusion protein in one of the following ways:
linking the amino terminus of an FGF21 fragment to the carboxy terminus of a GIPR antibody light chain via a peptide linker sequence (Linker): N'-R-Linker-FGF21-C';
linking the amino terminus of an FGF21 fragment to the carboxy terminus of a GIPR antibody heavy chain via a peptide linker sequence (Linker): N'-R-Linker-FGF21-C';
wherein N' represents the amino terminus of a polypeptide chain, C' represents the carboxy terminus of a polypeptide chain, FGF21 represents an FGF21 fragment, R is the amino acid sequence of the light or heavy chain of the GIPR antibody of any one of claims 1 to 29, and Linker represents a peptide linker sequence.

35. The FGF21 fusion protein of any one of claims 30 to 34, wherein the peptide linker sequence (Linker) comprises a full-length, partial, or repeated amino acid sequence independently selected from one of the following sequences: SEQ ID NO: 61, SEQ ID NO: 62, SEQ ID NO: 63, SEQ ID NO: 64, SEQ ID NO: 65, SEQ ID NO: 66, SEQ ID NO: 67 and SEQ ID NO: 68.

36. The FGF21 fusion protein of any one of claims 31 to 34, wherein the FGF21 fragment comprises an amino acid sequence independently selected from one of the following sequences: SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 115, SEQ ID NO: 116, SEQ ID NO: 117, SEQ ID NO: 118 and SEQ ID NO: 122.

37. The FGF21 fusion protein of any one of claims 30 to 36, wherein the FGF21 fragment comprises SEQ ID NO: 89 or SEQ ID NO: 118.

38. The fusion protein of any one of claims 30 to 37, wherein the fusion protein comprises a GIPR antibody, two FGF21 fragments, and a peptide linker sequence between the GIPR antibody and the FGF21 fragments, wherein the sequence of the fusion protein comprises an amino acid sequence independently selected from one of the following sequences:
a. an amino acid sequence of a light chain: SEQ ID NO: 103
b. an amino acid sequence of a modified heavy chain (a heavy chain, peptide linker, and FGF21 analog): SEQ ID NO: 104, SEQ ID NO: 105, SEQ ID NO: 106, SEQ ID NO: 119, SEQ ID NO: 120, SEQ ID NO: 121, SEQ ID NO: 123, SEQ ID NO: 124 and SEQ ID NO: 125.

39. A polynucleotide, encoding the GIPR antibody of any one of claims 1 to 29 or the FGF21 fusion protein of any one of claims 30 to 37.

40. A vector, comprising the polynucleotide of claim 39.

41. A host cell, comprising the vector of claim 40.

42. A pharmaceutical composition, comprising the GIPR antibody of any one of claims 1 to 29 or the FGF21 fusion protein of any one of claims 30 to 38, mixed with a pharmaceutically acceptable carrier.

43. Use of a pharmaceutical composition comprising the GIPR antibody of any one of claims 30 to 38 or the FGF21 fusion protein of any one of claims 30 to 37 in the preparation of a medicament for preventing or treating non-alcoholic fatty liver-related diseases.

44. Use of a pharmaceutical composition comprising the GIPR antibody of any one of claims 30 to 38 or the FGF21 fusion protein of any one of claims 30 to 37 in the preparation of a medicament for preventing or treating obesity and conditions associated with obesity.

45. Use of a pharmaceutical composition comprising the GIPR antibody of any one of claims 30 to 38 or the FGF21 fusion protein of any one of claims 30 to 37 in the preparation of a medicament for preventing or treating of Type 2 diabetes.

46. Use of a pharmaceutical composition comprising the GIPR antibody of any one of claims 30 to 38 or the FGF21 fusion protein of any one of claims 30 to 37 in the preparation of a medicament for simultaneously preventing or treating two or more conditions of non-alcoholic fatty liver-related diseases, obesity or Type 2 diabetes.

47. The use of any one of claims 43 to 46, wherein the pharmaceutical composition is used for intravenous or subcutaneous injection.
